(19) **Europäisches Patentamt**
**European Patent Office**
**Office européen des brevets**

(11) **EP 3 351 934 A1**

(12) **EUROPEAN PATENT APPLICATION**
published in accordance with Art. 153(4) EPC

(43) Date of publication:
**25.07.2018 Bulletin 2018/30**

(51) Int Cl.:
*G01N 33/497* (2006.01)        *G01N 33/48* (2006.01)
*G01N 33/574* (2006.01)

(21) Application number: **16850672.3**

(22) Date of filing: **28.09.2016**

(86) International application number:
**PCT/JP2016/004374**

(87) International publication number:
**WO 2017/056493 (06.04.2017 Gazette 2017/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **29.09.2015 JP 2015192241**

(71) Applicants:
• **Nissha Co., Ltd.**
  **Kyoto-shi, Kyoto 604-8551 (JP)**
• **Kyoto University**
  **Kyoto-shi, Kyoto 606-8501 (JP)**

(72) Inventors:
• **HANADA, Mariko**
  **Kyoto-shi**
  **Kyoto 604-8551 (JP)**
• **KODA, Hiroshi**
  **Kyoto-shi**
  **Kyoto 604-8551 (JP)**
• **KOMURA, Hajime**
  **Itami-shi**
  **Hyogo 664-0891 (JP)**
• **MORIMOTO, Satoshi**
  **Kyoto-shi**
  **Kyoto 604-8551 (JP)**

• **TOKUNO, Katsumi**
  **Kyoto-shi**
  **Kyoto 604-8551 (JP)**
• **TANAKA, Katsuyuki**
  **Kyoto-shi**
  **Kyoto 604-8551 (JP)**
• **ASHIDA, Noriko**
  **Kyoto-shi**
  **Kyoto 604-8551 (JP)**
• **KODAMA, Yuzo**
  **Kyoto-shi**
  **Kyoto 606-8501 (JP)**
• **CHIBA, Tsutomu**
  **Kyoto-shi**
  **Kyoto 606-8501 (JP)**
• **MIMA, Atsushi**
  **Kyoto-shi**
  **Kyoto 606-8501 (JP)**
• **TAKADA, Masahiro**
  **Kyoto-shi**
  **Kyoto 606-8501 (JP)**
• **TOI, Masakazu**
  **Kyoto-shi**
  **Kyoto 606-8501 (JP)**

(74) Representative: **Stöckeler, Ferdinand et al
Schoppe, Zimmermann, Stöckeler
Zinkler, Schenk & Partner mbB
Patentanwälte
Radlkoferstrasse 2
81373 München (DE)**

(54) **CANCER DEVELOPMENT RISK ASSESSMENT DEVICE, PROGRAM, AND METHOD FOR TESTING CANCER DEVELOPMENT RISK**

(57)    Provided is a cancer development risk assessment device (1) capable of determining the possibility of cancer development in a subject to be assessed in short time. The cancer development risk assessment device (1) includes a measurement unit (3) and a controller (4). The measurement unit (3) is configured to measure concentrations of a plurality of types of target components included in exhaled air collected from a subject to be assessed. The controller (4) is configured to calculate an assessment score correlated to the possibility of cancer being developed in the subject to be assessed based on measurement results of the concentrations obtained by the measurement unit (3).

EP 3 351 934 A1

# FIG. 1

**Description**

TECHNICAL FIELD

This invention relates to an assessment device for cancer development risk operable to determine the possibility of cancer being developed in a human subject and thereby provide helpful information in advance of cancer diagnosis, a program for the assessment device, and a testing method for cancer development risk.

BACKGROUND ART

In recent years, continuous increase of cancer-related mortality rates has been and still is a matter of serious concern. The mortality rates increase with delays in cancer detection, which calls for early detection of cancer.

Examples of cancer diagnostic approaches may include computer tomography, magnetic resonance imaging, X-ray examination, positron emission tomography, endoscopy, and blood marker tests.

These are, however, hardly non-invasive, complicated, and costly examinations, which are difficult to be exercised in general medical examinations. This may certainly be a factor for late detection of cancer.

Early detection of cancer may greatly benefit from non-invasive methods for determining the possibility of cancer development, if made available, which allows to readily determine whether thorough examinations are further necessary. In the meantime, there are ongoing attempts in many research institutions to determine the possibility of cancer development by testing exhaled air.

For example, Patent Literature 1 describes a method in which exhaled air is collected from human subjects to measure concentrations of the following components in the exhaled air; acetone, acetophenone, nitrogen oxide, propenal, phenol, benzaldehyde, 2-butanone, ethyl propionate, methylisobutenoate, and nonanal. In this method, lung cancer is determined upon detection of increases in concentration by 1 ppm to 5 ppm as compared to the recorded history of concentration measurement results.

However, the method described in Patent Literature 1 requires long-term collection of exhaled air from human subjects, which is a time-consuming and costly process.

CITATION LIST

PATENT LITERATURE

PATENT LITERATURE 1: JP 2009-518654 A

SUMMARY OF THE INVENTION

To address the issues of the known art, this invention provides an assessment device for cancer development risk operable to determine in short time the possibility of cancer being developed in a target subject, a program for the assessment device, and a testing method for cancer development risk.

An assessment device for cancer development risk according to this invention includes: a measurement unit configured to measure concentrations of a plurality of types of target components included in exhaled air collected from a subject to be assessed; and a controller configured to calculate an assessment score correlated to the possibility of cancer being developed in the subject based on measurement results of the concentrations obtained by the measurement unit.

A program according to this invention is for use in prompting a computer to effectuate functions of the controller provided in the assessment device for cancer development risk.

A testing method for cancer development risk according to this invention includes steps of: (a) collecting exhaled air from a subject to be assessed; (b) measuring concentrations of a plurality of types of target components included in the exhaled air; and (c) comparing an assessment score to a reference score based on a criterion that cancer is being developed in the subject to be assessed with a high possibility when the assessment score calculated from measurement results of the concentrations is greater than or equal to the reference score.

BRIEF DESCRIPTION OF THE DRAWINGS

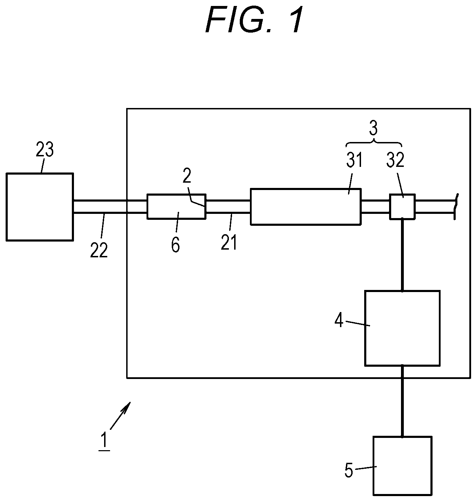

Fig. 1 is a schematic drawing of an assessment device for cancer development risk according to an embodiment of this invention.

3

Fig. 2 is a schematic drawing of a gas component adsorption tube used in an assessment method for cancer development risk according to an embodiment of this invention.

Fig. 3 is a schematic drawing of a gas component collecting device with the gas component adsorption tube illustrated in Fig. 2.

DESCRIPTION OF EMBODIMENTS

The description hereinafter given starts with the study findings which led the inventors to devise an assessment device and method for cancer development risk (hereinafter, assessment device, assessment method), and a testing method for cancer development risk (hereinafter, testing method).

A requirement in the method described in JP 2009-518654 A is long-term collection of exhaled air from a human subject, which is a time-consuming and costly process.

To address the issue, the inventors of this invention conducted the studies described below to provide an assessment device capable of determining in short time the possibility of cancer being developed in a human subject, and a program and an assessment method for the assessment device.

The conventional analyzing methods using exhaled air are only allowed to measure the concentrations of a limited range of components, because trace amounts of different components may be adhered to a device used to collect exhaled air.

The inventors analyzed multiple components including the components conventionally excluded from targets to be analyzed. Based on results thus obtained, the inventors found out differences between compositions of exhaled air collected from human subjects affected with cancer and of exhaled air collected from human subjects unaffected with cancer. Then, the inventors finally arrived at the finding that combining concentrations of two or more types of components included in exhaled air of a human subject may greatly contribute to assessment of the possibility of cancer being developed in the human subject, and they successfully completed this invention.

A further detailed description is given to such differences in composition of exhaled air between human subjects affected with cancer and unaffected with cancer elucidated by the present inventors.

The inventors analyzed exhaled air collected from a plurality of test subjects as described below. The test subjects included those diagnosed with breast cancer (hereinafter, breast cancer subjects), those diagnosed with pancreatic cancer (hereinafter, pancreatic cancer subjects), and those diagnosed with neither breast cancer nor pancreatic cancer (hereinafter, non-cancer subjects). The breast cancer subjects and the pancreatic cancer subjects may be collectively referred to as cancer subjects. The pancreatic cancer subjects and non-cancer subjects may be collectively referred to as non-breast cancer subjects. The breast cancer subjects and non-cancer subjects may be collectively referred to as non-pancreatic cancer subjects.

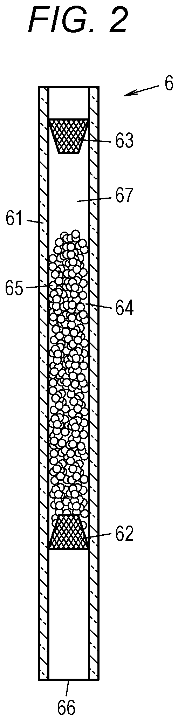

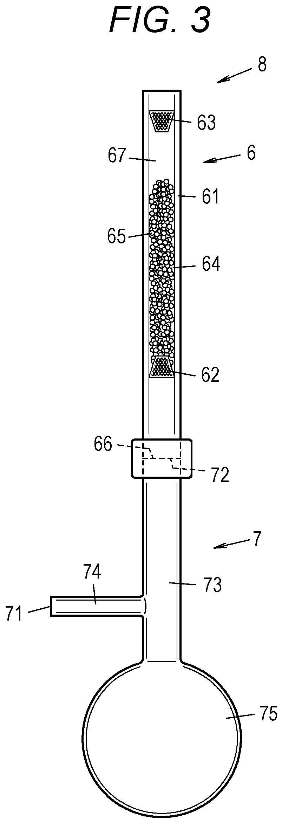

First, as adsorbent particles, activated carbon with an excellent capacity to adsorb components in exhaled air was selected. Then, a gas component collecting device with a gas component adsorption tube (manufactured by Nissha Co., Ltd.) was prepared. The gas component adsorption tube was filled with the adsorbent particles. Fig. 2 is a structural drawing of the gas component adsorption tube. Fig. 3 is a structural drawing of the gas component collecting device.

As illustrated in Fig. 2, a gas component adsorption tube 6 has an adsorption tube body 61 for passage of a gas sample, a pair of gas-permeable holders 62 and 63 disposed in the adsorption tube body 61, a containing space 64 between the paired holders 62 and 63, and a large number of adsorbent particles 65 filled in the containing space 64 to adsorb particular components in the gas sample. In the adsorption tube body 61 located at an upright position, a diffusion space 67 is formed in the containing space 64 between an aggregate of adsorbent particles 65 and the holder 63 above the particles.

As illustrated in Fig. 3, the gas component collecting device 8 has the gas component adsorption tube 6, and an introducing tool 7 for introducing the gas sample into the gas component adsorption tube 6. The introducing tool 7 has a non-linear introducing tube having openings at its both ends. Specifically, the introducing tool 7 has a main tube 73, a sub tube 74 diverging from the main tube 73, and a liquid receiver 75 at a lower end of the main tube 73. The sub tube 74 has an opening 71 which is an inlet for the gas sample to be introduced into the device. The main tube 73 has an opening 72, and a lower-end opening 66 of the adsorption tube body 61 in an upright position is coupled to the opening 72.

This gas component collecting device 8 was used to collect exhaled air from the test subjects. The test subjects were instructed to release exhaled air slowly in one breath, and the exhaled air was quickly collected into the gas component adsorption tube 6. Then, components included in the exhaled air were adsorbed to the adsorbent particles 65. From each test subject, 2L to 3L of exhaled air was collected.

Then, the gas component adsorption tube 6 was heated during constant supply of helium gas; carrier gas, into this adsorption tube 6 to release the adsorbed components from the adsorbent particles 65. The released components were sent to and analyzed in a gas chromatograph mass spectrometer.

Subsequent to the analysis by the gas chromatograph mass spectrometer, a multireactive, real-time mass spectrometer was further used to analyze the components.

In this manner, the inventors were able to identify a variety of types of components and their concentrations in exhaled air of each test subject. Then, the inventors extracted the components closely correlated to existence or non-existence of cancer development as described below.

The collected exhaled air was analyzed to detect various types of components and measure their concentrations. Any substances assumed to derive from the atmosphere and analyzers were removed from the detected components. Then, peak intensities of the remaining components in the chromatograph obtained by the gas chromatograph mass spectrometer were obtained. The measured values are peak intensities each indicating the mass of a component. The values of these peak intensities correlate to concentrations of the components in exhaled air. In this invention, measuring values correlated to the component concentrations is included in measuring the component concentrations, therefore, measuring peak intensities of the components is also included in measuring the component concentrations. The peak intensities were compared among the breast cancer subjects, pancreatic cancer subjects, and non-cancer subjects to identify the components in which significant differences were observed.

This identified the components that are closely correlated to existence or non-existence of pancreatic cancer but are remotely correlated to existence or non-existence of breast cancer; 1-methylpyrrolidine, acetaldehyde, acetone, methanol, dimethyl trisulfide, 2,4-dimethylhexane, 1-heptene, acetic acid methyl ester, 2-methyl-1,3-butadiene, 2-ethyl-1-hexanol, 3-methyl-3-buten-1-ol, acetonitrile, hexanal, hexanoic acid, 1-pentene, 5-methyl-1-heptene, 7,8-dioxabicyclo[3.2.1]oct-2-ene, menthol, propanol, dimethyl disulfide, 2-butene, 4-isopropenyltoluene, 1,2,4-trimethyl-benzene, and 2-propanol. The analyses using the multireactive, real-time mass spectrometer indicates close correlation of methyl mercaptan, hexanone, and dimethyl sulfide to existence or non-existence of pancreatic cancer development. The hexanone may be either one of or both of 2-hexanone and 3-hexanone. The group consisting of these components is hereinafter referred to as a group of pancreatic cancer markers.

The components that are closely correlated to existence or non-existence of breast cancer development but are remotely correlated to existence or non-existence of pancreatic cancer development were further identified; 2-methyl-butyric acid, 1-heptanol, 2-methyl-1-propanol, cyclohexanone, 1-pentanol, 4-ethylcyclohexanol, nitrosomethylurethane, decane, dodecane, 2,2,4-trimethylhexane, nonane, 2,4-dimethyl-heptane, 1-octene, butylbenzene, ethylacetate, o-xylene, β-phellandrene, 2-propenal, D-limonene, 1-methoxy-2-propanol, octanal, 1,3-dichloro-benzene, 1-decanol, $\alpha,\alpha$-dimethyl-β-phenylethyl=acetate, 2-methyl-phenol, ethylacetate, DL-alanyl-L-alanine, and 3,3-dimethyl-(3H)indazole. The group consisting of these components is hereinafter referred to as a group of breast cancer markers.

The components closely correlated to existence or non-existence of breast cancer development and pancreatic cancer development both were also identified; 6-methyl-5-hepten-2-one, benzaldehyde, heptanal, acetic acid, 2-methyl-propionic acid, decanal, phenol, butanal, butyric acid, 1-octen-3-ol, 1-propanol, acetoin, propionic acid, 2,3-butanedione, mercaptoacetone, trimethylamine, acetophenone, 2-methylfuran, 1,2-ethanediol, 1-butanol, n-hexane, 2,4-bis(1,1-dimethylethyl)-phenol, 3-methyl-1-butanol, nonanal, 1-hexanol, 2-butanone, and carbon disulfide. These components found to closely correlate to existence or non-existence of breast and pancreatic cancers both may be reasonably assumed to closely correlate to existence or non-existence of other cancers as well as breast and pancreatic cancers. The group consisting of these components is hereinafter referred to as a group of common cancer markers.

Then, degrees of sensitivity and specificity were delivered for each of these components. In assessment of the possibility of cancer development, the sensitivity is indicative of the ratio of those who are determined to be highly likely to have cancer to all of cancer subjects, and the specificity is indicative of the ratio of those who are not determined to be highly likely to have cancer to all of non-cancer subjects. In assessment of the possibility of breast cancer development, the sensitivity is indicative of the ratio of those who are determined to be highly likely to have breast cancer to all of breast cancer subjects, and the specificity is indicative of the ratio of those who are not determined to be highly likely to have breast cancer to all of non-breast cancer subjects. In assessment of the possibility of pancreatic cancer development, the sensitivity is indicative of the ratio of those who are determined to be highly likely to have pancreatic cancer to all of pancreatic cancer subjects, and the specificity is indicative of the ratio of those who are not determined to be highly likely to have pancreatic cancer to all of non-pancreatic cancer subjects.

The components capable of effectively increasing the sensitivity, the components capable of effectively increasing the specificity, and the components capable of effectively increasing the sensitivity and specificity in total in the process of assessing the possibility of cancer development were extracted from the components included in the group of pancreatic cancer markers, the components included in the group of breast cancer markers, and the components included in the group of common cancer markers. The following components were found to be capable of effectively increasing the sensitivity or specificity, or the sensitivity and specificity in total; 1-methylpyrrolidine, carbon disulfide, acetaldehyde, 2-methyl-butyric acid, acetone, 6-methyl-5-hepten-2-one, 2-methyl-propionic acid, decanal, benzaldehyde, acetic acid, butyric acid, 1-propanol, propionic acid, 1-heptanol, heptanal, 2-methyl-1-propanol, phenol, trimethylamine, 1-octen-3-ol, mercaptoacetone, 2-butanone, acetophenone, 2-methylfuran, acetoin, 2,3-butanedione, methanol, cyclohexanone, dimethyl trisulfide, 2,4-dimethylhexane, 1-pentanol, n-hexane, 1-heptene, 4-ethylcyclohexanol, acetic acid methyl ester, nitrosomethylurethane, 2-methyl-1,3-butadiene, decane, 3-methyl-1-butanol, dodecane, 2-ethyl-1-hexanol, 2,2,4-trimethylhexane, 3-methyl-3-buten-1-ol, acetonitrile, 2,4-bis(1,1-dimethylethyl)-phenol, hexanal, nonane, nonanal, butanal, 2,4-dimethylheptane, hexanoic acid, 1-octene, 1,2-ethanediol, 1-pentene, butylbenzene, 5-methyl-1-heptene, ethylacetate, 7,8-dioxabicyclo[3.2.1]oct-2-ene, o-xylene, 1-hexanol, β-phellandrene, menthol, 2-propenal, propanal, D-limonene, dimethyl disulfide, 1-methoxy-2-propanol, 2-butene, octanal, 4-isopropenyltoluene, 1,3-dichlorobenzene, 1,2,4-trimethylbenzene, and 1-butanol. These components were thus found to be particularly useful for assessment of the possibility of cancer development. According to analyses using the multireactive, real-time mass spectrometer, methyl mercaptan and dimethyl sulfide were also found to be particularly useful for assessment of the possibility of cancer development. The group consisting of these components is hereinafter referred to as a group of high-accuracy cancer markers.

The substances capable of effectively increasing the sensitivity or specificity, or the sensitivity and specificity in total in the process of assessing the possibility of breast cancer development were extracted from the components included in the group of breast cancer markers and the components included in the group of common cancer markers. The following components were found to be capable of effectively increasing the sensitivity or specificity, or the sensitivity and specificity in total; benzaldehyde, heptanal, acetic acid, 2-methyl-butyric acid, 6-methyl-5-hepten-2-one, 2-methyl-propionic acid, decanal, phenol, butanal, butyric acid, 1-octen-3-ol, 1-heptanol, 2-methyl-1-propanol, 1-propanol, acetoin, propionic acid, 2-butanone, 2,3-butanedione, mercaptoacetone, trimethylamine, acetophenone, cyclohexanone, 1,2-ethanediol, 1-pentanol, 1-butanol, 4-ethylcyclohexanol, nitrosomethylurethane, decane, dodecane, 2,2,4-trimethylhexane, n-hexane, 2,4-bis(1,1-dimethylethyl)-phenol, nonane, 3-methyl-1-butanol, 2,4-dimethylheptane, 1-octene, 2-methylfuran, nonanal, butylbenzene, ethylacetate, o-xylene, β-phellandrene, 2-propenal, D-limonene, 1-methoxy-2-propanol, octanal, and 1,3-dichlorobenzene. The group consisting of these components is hereinafter referred to as a group of high-accuracy breast cancer markers.

The substances capable of effectively increasing the sensitivity or specificity, or the sensitivity and specificity in total in the process of assessing the possibility of pancreatic cancer development were extracted from the components included in the group of pancreatic cancer markers and the components included in the group of common cancer markers. The following components were found to be capable of effectively increasing the sensitivity or specificity, or the sensitivity and specificity in total; 1-methylpyrrolidine, carbon disulfide, acetaldehyde, acetone, 2-methyl-propionic acid, decanal, benzaldehyde, acetic acid, butyric acid, 1-propanol, propionic acid, heptanal, phenol, trimethylamine, 1-octen-3-ol, mercaptoacetone, acetophenone, 2-methylfuran, 6-methyl-5-hepten-2-one, acetoin, 2,3-butanedione, methanol, dimethyl trisulfide, 2,4-dimethyl-hexane, n-hexane, 1-heptene, acetic acid methyl ester, 2-methyl-1,3-butadiene, 3-methyl-1-butanol, 2-ethyl-1-hexanol, 3-methyl-3-buten-1-ol, acetonitrile, hexanal, nonanal, butanal, hexanoic acid, 1,2-ethanediol, 2,4-bis(1,1-dimethylethyl)-phenol, 1-pentene, 5-methyl-1-heptene, 7,8-dioxabicyclo[3.2.1]oct-2-ene, 1-hexanol, menthol, propanal, dimethyl disulfide, 2-butene, 4-isopropenyltoluene, 1,2,4-trimethylbenzene, and 1-butanol. According to analyses using the multireactive, real-time mass spectrometer, dimethyl sulfide was also found to be capable of effectively increasing the sensitivity or specificity, or the sensitivity and specificity in total. The group consisting of these components is hereinafter referred to as a group of high-accuracy pancreatic cancer markers.

Then, substances particularly effective for assessment of the possibility of cancer development were extracted from the components included in the group of high-accuracy cancer markers. The following components were found to be capable of effectively increasing the sensitivity or specificity, or the sensitivity and specificity in total and also particularly useful for assessment of the possibility of cancer development; 1-methylpyrrolidine, 2-methyl-propionic acid, benzaldehyde, acetic acid, butyric acid, phenol, trimethylamine, 1-octen-3-ol, mercaptoacetone, acetophenone, acetoin, carbon disulfide, 2-methyl-butyric acid, 6-methyl-5-hepten-2-one, decanal, propionic acid, heptanal, 2-methylfuran, 2,3-butanedione, nonanal, butanal, and hexanoic acid. Also, methyl mercaptan, dimethyl sulfide, and dimethyl trisulfide were also found to be very effective. The group consisting of these components is hereinafter referred to as a group of most-relevant cancer markers.

Yet, whatever the components are used, it may still be difficult to determine the possibility of cancer development with accuracy high enough for practical use, not to speak of difficulty in determining with high accuracy the possibility of a specific type of cancer being developed, such as breast cancer or pancreatic cancer.

The inventors focused their efforts on improvement of accuracy in assessment of the possibility of cancer development and any effective methods for determining the possibility of a specific type(s) of cancer being developed. Then, the inventors came up with the idea of selecting two or more types of components and combining measurement results of their concentrations in exhaled air, and found such combined measurement results to more closely correlate to existence or non-existence of cancer development than the concentration measurement result of one type of component. Thus combining the measurement results was also found to be useful for selectively determining whether specific types of cancer are possibly developed.

The studies further led the inventors to find combinations of the components that may allow the possibility of cancer development to be very accurately determined in determining the possibility of cancer development.

<Assessment method>

An assessment method according to an embodiment of this invention includes steps of: (a) collecting exhaled air from a subject to be assessed (hereinafter, target subject); (b) measuring concentrations of a plurality of types of target components in the exhaled air; and (c) calculating an assessment score correlated to the possibility of cancer being developed in the target subject based on measurement results of the concentrations. For example, the possibility of cancer development may be determined as higher with greater or smaller values of the assessment score.

In the step (c), the possibility of cancer being developed in the target subject may be determined as high when the assessment score is equal to a certain value or within a certain range of values. When, for example, the possibility of cancer development is determined as higher with greater values of the assessment score, the target subject may be determined to be highly likely to have cancer when the assessment score is greater than or equal to a predetermined reference score. The reference score may be decided based on analysis results of the exhaled air collected from a plurality of test subjects. In the step (c), the possibility of cancer being developed in the target subject may be determined as low when the assessment score is neither equal to a certain value nor within a certain range of values.

The assessment method according to this embodiment may allow assessment of the possibility of cancer being developed in a target subject by simply analyzing exhaled air of the target subject without having to perform long-term monitoring. The assessment method according to this embodiment may allow highly reliable assessment by using concentration measurement results of two or more types of target components.

This embodiment using concentration measurement results of two or more types of target components for assessment may provide reliable assessment of the possibility of breast cancer being developed in a target subject and the possibility of pancreatic cancer being developed in a target subject.

The plural types of target components may include at least two types of components selected from the group of common cancer markers. In this instance, the possibility of cancer being developed in a target subject may be determined irrespective of types of cancer. The plural types of target components may solely consist of at least two types of components selected from the group of common cancer markers alone.

The plural types of target components may include at least two types of components selected from the group of high-accuracy cancer markers. In this instance, the possibility of cancer being developed in a target subject may be determined by a high sensitivity and a high specificity irrespective of types of cancer. The plural types of target components may solely consist of at least two types of components selected from the group of high-accuracy cancer markers alone. The plural types of target components may further include at least one type of component selected from the components that are included in the group of common cancer markers but are not included in the group of high-accuracy cancer markers.

The plural types of target components may include at least two types of components selected from the group of breast cancer markers. In this instance, the possibility of breast cancer being developed in a target subject may be determined. The plural types of target components may solely consist of any one(s) of the components selected from the group of breast cancer markers alone.

The plural types of target components may include at least two types of components selected from the group of high-accuracy breast cancer markers. In this instance, the possibility of breast cancer being developed in a target subject may be determined by a high sensitivity and a high specificity. The plural types of target components may solely consist of any one(s) of the components selected from the group of high-accuracy breast cancer markers. The plural types of target components may further include at least one type of component selected from the components that are included in the group of breast cancer markers but are not included in the group of high-accuracy breast cancer markers.

The plural types of target components may include at least two types of components selected from the group of pancreatic cancer markers. In this instance, the possibility of pancreatic cancer being developed in a target subject may be determined. The plural types of target components may solely consist of at least two types of components selected from the group of pancreatic cancer markers alone.

The plural types of target components may include at least two types of components selected from the group of high-accuracy pancreatic cancer markers. In this instance, the possibility of pancreatic cancer being developed in a target subject may be determined by a high sensitivity and a high specificity. The plural types of target components may solely consist of any one(s) of the components selected from the group of high-accuracy pancreatic cancer markers. The plural types of target components may further include at least one type of component selected from the components that are included in the group of pancreatic cancer markers but are not included in the group of high-accuracy pancreatic cancer markers.

The plural types of target components may include at least two types of components selected from the group of most-relevant cancer markers. In this instance, the possibility of cancer being developed in a target subject may be determined by a high sensitivity and a high specificity irrespective of types of cancer. The plural types of target components may solely consist of at least two types of components selected from the group of most-relevant cancer markers alone. The plural types of target components may further include at least one type of component selected from the components that are included in the group of common cancer markers but are not included in the group of most-relevant cancer markers.

The plural types of target components may preferably include at least one type of component selected from the group consisting of benzaldehyde, 2-methyl-propionic acid, mercaptoacetone, trimethylamine, 1-methylpyrrolidine, acetophenone, 2-methylfuran, 1-octen-3-ol, propionic acid, acetoin, 2-butanone, 1,2-ethanediol, 2,3-butanedione, acetaldehyde, 2-methyl-butyric acid, 1-decanol, 3-methyl-1-butanol, 1-hexanol, 1-heptanol, 2-propenal, ethylacetate, and 3,3-dimethyl-(3H)indazole. Using any one(s) of these components may particularly improve the accuracy of assessing the possibility of cancer development when different types of target components are used for assessment, as described in this embodiment. In assessment of the possibility of breast cancer development, particularly, the target components may preferably include at least one type of component selected from the group consisting of 2-methyl-butyric acid, 1-decanol, 1-heptanol, 2-propenal, ethylacetate, 3,3-dimethyl-(3H)indazole, benzaldehyde, 2-methyl-propionic acid, mercaptoacetone, trimethylamine, acetophenone, 2-methylfuran, 1-octen-3-ol, propionic acid, acetoin, 2-butanone, 1,2-ethanediol, 2,3-butanedione, 3-methyl-1-butanol, and 1-hexanol. In assessment of the possibility of pancreatic cancer development, the target components may preferably include at least one type of component selected from the group consisting of 1-methylpyrrolidine, acetaldehyde, benzaldehyde, 2-methyl-propionic acid, mercaptoacetone, trimethylamine, acetophenone, 2-methylfuran, 1-octen-3-ol, propionic acid, acetoin, 2-butanone, 1,2-ethanediol, 2,3-butanedione, 3-methyl-1-butanol, and 1-hexanol.

In the step (c) of the assessment method according to this embodiment, the concentration measurement results of different types of target components are compared to thresholds respectively set for the target components in advance of the assessment score being calculated. Then, the assessment score is calculated based on the result of comparison.

For example, the concentration of a target component is the ratio of the target component included in exhaled air collected from a subject to the whole exhaled air.

The concentration of a target component may be a relative concentration calculated on the basis of the ratio of any one component but the target components included in exhaled air (hereinafter, reference component). The relative concentration may be a value obtained when the ratio of a target component included in exhaled air to the whole exhaled air is divided by the ratio of the reference component included in the exhaled air to the whole exhaled air. When the concentration of a target component is the relative concentration thus calculated, the possibility of cancer development may be more accurately assessed. The reference component may preferably be selected from components discharged from human body in less variable amounts. Specific examples of the reference component may preferably include water vapor and 2-butene. With such a reference component, the possibility of cancer development may be particularly accurately assessed.

The assessment score may be calculated otherwise in the step (c) of the assessment method according to this embodiment. For example, the concentrations of different types of target components may be standardized to calculate standardized values of the respective target components. The assessment score may be calculated based on a result of comparison of the standardized values to thresholds respectively set for the target components. In this instance, the possibility of cancer development may be even more accurately assessed.

The standardized value may be calculated by Formula (2).

$$A_i = \frac{(a_i - x_i)}{y_i} \quad ...(2)$$

In Formula (2), $a_i$ is the concentration measurement result of an i(th) target component, $x_i$ is a mean value of concentrations of the i(th) components included in exhaled air collected from a plurality of test subjects, $y_i$ is a standard deviation of concentrations of the i(th) components in exhaled air collected from the plurality of test subjects, and $A_i$ is a standardized value of the i(th) component as described earlier, where $x_i$ and $y_i$ are decided from analysis results of exhaled air collected from the plurality of test subjects.

In the case of $a_i$ being the relative concentration measurement result of the i(th) target component when the standardized value is calculated, the possibility of cancer development may be very accurately assessed.

The thresholds are decided based on the analysis results of exhaled air collected from a plurality of test subjects. The exhaled air collected from a plurality of test subjects is analyzed as described earlier. The threshold is a value that divides the concentration distribution of a target component in exhaled air collected from a plurality of test subjects into a set of concentrations with a higher possibility of cancer development (hereinafter, positive set) and a set of concentrations with a lower possibility of cancer development (hereinafter, negative set). The threshold may be decided so as to include, in the positive set, preferably 20% or more of subjects affected with cancer, more preferably 50% or more of subjects affected with cancer, or even more preferably 80% or more of subjects affected with cancer. The threshold may be decided so as to include, in the negative set, preferably 75% or more of subjects unaffected with cancer, more preferably 85% or more of subjects unaffected with cancer, or even more preferably 95% or more of subjects unaffected with cancer.

In assessment of the possibility of cancer development irrespective of types of cancer, the positive set includes a relatively high percentage of cancer subjects, while the negative set includes a relative low percentage of cancer subjects. The threshold may be decided for distinction between the positive set and the negative set. In this instance, the threshold may be a value decided in accordance with the concentration distribution of a target component, examples of which include a third quartile or a median calculated from the concentration distribution of a target component in exhaled air of non-cancer subjects, or a median calculated from the concentration distribution of a target component in exhaled air of all of test subjects.

In assessment of the possibility of breast cancer, the positive set includes a relatively high percentage of breast cancer subjects, while the negative set includes a relative low percentage of breast cancer subjects. The threshold may be decided for distinction between the positive set and the negative set. In this instance, the threshold may be a value decided in accordance with the concentration distribution of a target component, examples of which include a third quartile or a median calculated from the concentration distribution of a target component in exhaled air of non-breast cancer subjects, or a median calculated from the concentration distribution of a target component in exhaled air of all of test subjects.

In assessment of the possibility of pancreatic cancer, the positive set includes a relatively high percentage of pancreatic cancer subjects, while the negative set includes a relative low percentage of pancreatic cancer subjects. The threshold may be decided for distinction between the positive set and the negative set. In this instance, the threshold may be a value decided in accordance with the concentration distribution of a target component, examples of which include a third quartile or a median calculated from the concentration distribution of a target component in exhaled air of non-pancreatic cancer subjects, or a median calculated from the concentration distribution of a target component in exhaled air of all of test subjects.

The threshold for concentrations may be decided in the manner thus described, which may also be applicable to a threshold for concentration standardized values.

Calculation of the assessment score may start with comparing the concentration measurement results of target components or their standardized values to the thresholds. Then, a predetermined value when the measurement result is included in the positive set is decided and used as the score of each target component, and another predetermined value when the measurement result is included in the negative set is further decided. For example, the score when the measurement result is included in the positive set may be "1 point", and the score when the measurement result is included in the negative set may be "0 point".

Any target component that leads to a high sensitivity, a high specificity, or a high sensitivity and specificity in total may be assessed as greatly contributing to the process of determining the possibility of cancer development. To decide the scores of respective target components using thresholds, levels of contribution to the process of determining existence or non-existence of cancer development may be taken into account. This may improve the accuracy of determining the possibility of cancer development. When, for example, a plurality of types of target components differ in sensitivity or specificity, or sensitivity and specificity in total, indicating differences between their levels of contribution when determining the possibility of cancer development, such target components may be scored differently in accordance with their levels of contribution. When the sensitivity is emphasized over the other to determine the possibility, the levels of contribution are decided according to differences in sensitivity. When the specificity is emphasized over the other to determine the possibility, the levels of contribution are decided according to differences in specificity. When the sensitivity and specificity are both emphasized to determine the possibility, the levels of contribution are decided according to the total of sensitivity and specificity. As for any target component with a higher level of contribution than the others, for example, the score when the measurement result is included in the positive set is set to "2 points", and the score when the measurement result is included in the negative set is set to "0 point". As for any target component with a lower level of contribution than the others, for example, the score when the measurement result is included in the positive set is set to "1 point", and the score when the measurement result is included in the negative set is set to "0 point".

When the assessment score is calculated from the points thus decided for the target components, the scores in total, for example, are used as the assessment score. The assessment score may be appropriately calculated otherwise. For example, the assessment score may be a multiplied result of all of the scores. In this instance, higher assessment score may be assessed as indicating a higher possibility of cancer development.

Taking into account difference levels of contribution between the target components to the process of determining the possibility of cancer development, for example, the target components may be divided into different groups depending on their levels of contribution, and the score of each group may be decided based on acquired points of target components in the group according to a predetermined rule adapted for their levels of contribution to decide the assessment score based on the scores of these groups. The target components may be divided into, for example, a first group with more contribution to the process of determining the possibility of cancer development, and a second group with less contribution to the process of determining the possibility of cancer development. In this instance, the score of the first group may be decided as the points in total acquired by the target components included in the first group. The score of the second group may be decided as 1 point when the points in total acquired by the target components included in the second group are greater than or equal to a predetermined score (for example, 1 point), and may be decided as 0 point when the points in total are 0 point. Then, the scores in total of these groups may be decided as the assessment score.

The possibility of cancer being developed in a target subject may be determined as high when the assessment score is equal to a certain value or within a certain range of values. Otherwise, the possibility of cancer being developed in the target subject may be determined as low. When, for example, the assessment score is decided so that the possibility of cancer development is higher with greater values of the assessment score, a target subject may be determined to be highly likely to have cancer when the assessment score is greater than or equal to a reference score. The possibility of cancer being developed in the target subject may be determined as low when the assessment score is less than the reference score. The reference score is decided and set, so that one or both of sensitivity and specificity are high, based on analysis results of exhaled air collected from a plurality of test subjects. This may allow the possibility of cancer development to be determined with high accuracy.

The assessment method may include a plurality of assessments that respectively use different groups of target components. In this instance, the possibility of cancer development may be very accurately determined by combining, for example, an assessment that excels in sensitivity and an assessment that excels in specificity.

The assessment method may include a first assessment that allows determination of the possibility of cancer development with a relatively high specificity and a second assessment that allows determination of the possibility of cancer development with a relatively high sensitivity. In this instance, the assessment method may start with the first assessment. Then, the possibility of cancer being developed in a target subject may be determined as high when the assessment score obtained then is equal to a certain value or within a certain range of values. Otherwise, the assessment method, without determining the possibility at this stage, proceeds to the second assessment. In the second assessment, the possibility of cancer being developed in the target subject may be determined as high when the assessment score obtained then is equal to a certain value or within a certain range of values. Otherwise, the possibility of cancer development may be determined as low.

The assessment method may include a first assessment that allows determination of the possibility of cancer development with a relatively high sensitivity and a second assessment that allows determination of the possibility of cancer development with a relatively high specificity. In this instance, the assessment method may start with the first assessment. When the assessment score obtained then is equal to a certain value or within a certain range of values, the assessment method, without determining the possibility at this stage, proceeds to the second assessment. Otherwise, the possibility of cancer development may be determined as low. In the second assessment, the possibility of cancer being developed in the target subject may be determined as high when the assessment score obtained then is equal to a certain value or within a certain range of values. Otherwise, the possibility of cancer development may be determined as low.

To calculate the assessment score in the step (c) of the assessment method according to this embodiment, the concentrations of different types of target components may be standardized to calculate standardized values of the respective target components, and the assessment score may be calculated based on the standardized values and their levels of contribution. The level of contribution of a standardized value described herein refers to a quantified value of correlation between the standardized value and existence or non-existence of cancer development. The level of contribution is decided for each target component from analysis results of exhaled air collected from a plurality of test subjects. This may allow the possibility of cancer development to be determined with particularly high accuracy.

The inventors' analysis results on exhaled air collected from a plurality of test subjects revealed close correlation between combination of two or more types of target components and existence or non-existence of cancer development, which was found to be useful for determining the possibility of cancer development with very high accuracy. Specifically, when the standardized values of different types of target components and their levels of contribution are used to determine the possibility of cancer development, combination of two or more of the standardized values and a level of contribution of the combination may be further used to calculate the assessment score. The possibility of cancer development may be accordingly determined with very high accuracy. Specifically, the assessment score may be calculated based on the standardized values and their levels of contribution and also based on combination of two or more of the standardized values and the level of contribution of the combination.

A specific example of the method for determining the possibility of cancer development using the standardized values and their levels of contribution is hereinafter described. The assessment score may be calculated by Formula (1).

$$S = \sum_{i=1}^{n} \sum_{j=1}^{n} \frac{k_{ij} A_i A_j}{n} \quad \ldots (1)$$

In Formula (1), n is the number of types of target components, and, among the target components numbered 1st to n(th), $A_i$ is the standardized value of an i(th) target component, $A_j$ is the standardized value of a j(th) target component, $k_{ij}$ is the level of contribution of combination of the standardized values of the i(th) and j(th) target components, and S is the assessment score. Where i = j, $k_{ij}$ is the level of contribution of a squared result of the standardized value of a target component (i.e., level of contribution of the standardized value of one type of target component). Where i ≠ j, $k_{ij}$ is the level of contribution of a multiplied result of the standardized values of two types of target components (i.e., level of contribution of combination of the standardized values of two types of target components).

The standardized value may be calculated by Formula (2).

In this formula, $k_{ij}$ is decided based on the analysis results of exhaled air collected from the test subjects.

For example, $k_{ij}$ may be decided in a manner described below. The concentrations of different types of target components in exhaled air collected from a plurality of test subjects are standardized to obtain a distribution of standardized values of the target components. Based on the obtained result, a correlation matrix between the target components as to existence or non-existence of cancer development and an inverse matrix thereof are calculated, based on which the value of $k_{ij}$ and Formula (1) may be derived from the formula of S = YAYT/n. In this formula, Y is a vector value representing a matrix value of concentration standardized values Y1, Y2, ... of the target components, A is the inverse matrix, and n is the number of types of target components. Assuming that there are two groups; a group of cancer subjects and a group of non-cancer subjects, the assessment score may be a Mahalanobis' generalized distance equivalent to a distance of cancer subjects from the center of the group of non-cancer subjects.

When Formula (1) is employed, three or more types of target components may preferably be used. In this instance, the possibility of cancer being developed in a target subject may be determined by a high sensitivity and a high specificity.

By employing Formula (1), the possibility of breast cancer being developed in a target subject may be determined by a high sensitivity and a high specificity when different types of target components include at least one type of component selected from the group of breast cancer markers. Further, the possibility of breast cancer being developed in a target subject may be determined by a higher sensitivity and a hither specificity when different types of target components include at least one type of component selected from the group of high-accuracy breast cancer markers.

By employing Formula (1), the possibility of pancreatic cancer being developed in a target subject may be determined by a high sensitivity and a high specificity when different types of target components include at least one type of component selected from the group of pancreatic cancer markers. Further, the possibility of pancreatic cancer being developed in a target subject may be determined by a higher sensitivity and a higher specificity when different types of target components include at least one type of component selected from the group of high-accuracy pancreatic cancer markers.

When Formula (1) is employed, the plural types of target components may preferably include three or more types of components selected from the group consisting of butanal, butyric acid, 2-methyl-butyric acid, heptanal, 2-methyl-propionic acid, acetone, carbon disulfide, n-hexane, phenol, propionic acid, 2-methyl-1,3-butadiene, 3-methyl-1-butanol, 1-heptanol, 5-methyl-1-heptene, 1-pentanol, 1-pentene, 2,3-butanedione, acetaldehyde, acetoin, 4-methylbenzaldehyde, butylbenzene, decanal, decane, carbon dioxide, 2,4-dimethyl-heptane, isopropyl alcohol, nonanal, octanal, 2,3-dimethyl-pentane, and undecane. The plural types of target components may more preferably include all of the components included in this group. In this instance, the possibility of breast cancer being developed in a target subject may be determined by a particularly high sensitivity and specificity.

When Formula (1) is employed, the plural types of target components may preferably include three or more types of components selected from the group consisting of (2-aziridinylethyl)amine, 3,3-dimethyl-(3H)indazole, 1-pentanol, 2-butene, 3-methyl-2-pentanone, 5-methoxymethoxyhexa-2,3-diene, acetic acid, acetonitrile, butanal, 2-methyl-butane, carbon disulfide, [1S-(1α,2β,4β)]-2-chloro-4-methyl-1-(1-methylethyl)-cyclohexane, cyclohexane, diallyl carbonate, 4,6-dimethyldodecane, 2,5-dimethyl-furan, glycol aldehyde dimer, heptanal, 2-methyl-heptane, (S)-1-alanine ethylamide, octane, 2,6,6-trimethyl-octane, 2-ethylhexylhexylester oxalic acid, 2,4-bis(1,1-dimethylethyl)-phenol, 2-methyl-phenol, propionic acid, 2-methyl-propionic acid, styrene, tetrahydrofuran, and β-phellandrene. The plural types of target components may more preferably include all of the components included in this group. In this instance as well, the possibility of breast cancer being developed in a target subject may be determined by a particularly high sensitivity and specificity.

When Formula (1) is employed, the plural types of target components may preferably include three or more types of components selected from the group consisting of decanal, 2-methyl-propionic acid, carbon disulfide, acetone, 1-propanol, 2-methyl-1,3-butadiene, acetaldehyde, acetoin, carbon dioxide, nonanal, 1,2-ethanediol, 1-butanol, 3-methyl-1-butanol, 1-hexanol, 2-ethyl-1-hexanol, 1-octene, 2,3-butanedione, acetic acid methyl ester, benzene, butyric acid, ethanol, ethylbenzene, eucalyptol, heptanal, o-cymene, oxygen, phenol, 1-(methylthio)-propane, propionic acid, and undecane. The plural types of target components may more preferably include all of the components included in this group. In this instance, the possibility of pancreatic cancer being developed in a test subject may be determined by a particularly high sensitivity and specificity.

When Formula (1) is employed, the plural types of target components may preferably include three or more types of components selected from the group consisting of 1,2-ethanediol, decanal, mercaptoacetone, heptanal, 6-methyl-5-hepten-2-one, cyclohexane, benzaldehyde, 1-pentanol, decane, 2,2,4-trimethyl-hexane, dodecane, 2-methyl-butyric acid, 1-butanol, n-hexane, 1-octen-3-ol, 4-ethylcyclohexanol, carbon disulfide, 2,4-bis(1,1-dimethylethyl)-phenol, nitrosomethylurethane, and acetoin. The plural types of target components may more preferably include all of the components included in this group. In this instance, the possibility of breast cancer being developed in a target subject may be determined by a particularly high sensitivity and specificity.

When Formula (1) is employed, plural types of target components may preferably include three or more types of components selected from the group consisting of 2-methyl-1,3-butadiene, 1-butanol, 3-methyl-1-butanol, 1-hexanol, 2-ethyl-1-hexanol, 1-propanol, 3-methyl-3-buten-1-ol, acetaldehyde, acetic acid, acetic acid methyl ester, acetone, acetonitrile, benzaldehyde, butyric acid, hexanal, isopropyl alcohol, methanol, nonanal, propanal, and 2-methyl-propionic acid. The plural types of target components may more preferably include all of the components included in this group. In this instance, the possibility of pancreatic cancer being developed in a target subject may be determined by a particularly high sensitivity and specificity.

In this embodiment, the assessment score may be calculated by Formula (3).

$$S = \sum_{i=1}^{n} A_i \times \alpha_i \quad ...(3)$$

In Formula (3), n is the number of types of target components, and, among the target components numbered 1st to n(th), $A_i$ is the concentration standardized value of an i(th) target component, and $\alpha_i$ is the level of contribution of the standardized value of the i(th) target component. As described earlier, the level of contribution of a standardized value described herein refers to a quantified value of correlation between the standardized value and existence/non-existence of cancer development. The level of contribution is decided for each target component from analysis results of exhaled air collected from a plurality of test subjects. In this formula, S is the assessment score. The standardized value may be calculated by Formula (2). In this instance, the possibility of cancer development may be more accurately assessed. When the standardized value is the standardized value of a relative concentration, i.e., $a_i$ in Formula (2) is the relative concentration of the i(th) target component, the possibility of cancer development may be even more accurately assessed. Further, a plurality of separate processing steps may be unnecessary, unlike comparison of the different target components to their thresholds. Hence, the standardized value may be simply multiplied by the level of contribution and added to obtain the assessment score.

In Formula (3), the value of $\alpha_i$ may be 1. With such a value of $a_i$, assessment of the possibility of cancer development may be improved in accuracy.

In Formula (3), $\alpha_i$ may be either a positive number or a negative number. According to this formula, assessment of the possibility of cancer development may be further improved in accuracy by assigning a positive number to $\alpha_i$ corresponding to a target component assessed as presenting a higher possibility of cancer development at a higher concentration in exhaled air, and by assigning a negative number to $\alpha_i$ corresponding to a target component assessed as presenting a lower possibility of cancer development at a higher concentration in exhaled air.

Regardless of whether Formula (3) is employed, the level of contribution may be either one of a positive number or a negative number.

In this embodiment, the concentrations of a plurality of types of target components included in exhaled air collected from a target subject are measured to assess and determine the possibility of cancer development. Then, the assessment score correlated to the possibility of cancer being developed in a subject to be assessed is calculated based on measurement results of the concentrations. As described so far, specific examples of the assessment score calculation method may include the threshold-based method and the standardized value-based method. These assessment score calculation method, however, are not limited thereto.

In this embodiment, the thresholds used to assess the possibility of cancer development and the reference score used to determine the possibility of cancer development may preferably be decided and set, so that the specificity and sensitivity in total are greater than or equal to 120%. The thresholds and the reference score may be optionally decided in accordance with purposes of assessing and determining the possibility of cancer development. When, for example, a subject determined as having a high possibility of cancer development undergoes a relatively simple examination for diagnosis of existence or non-existence of cancer development, the thresholds and the reference score may preferably be decided so as to attain a higher sensitivity. When, for example, a subject determined as having a high possibility of cancer development undergoes a physically demanding examination such as biopsy, the thresholds and the reference score may preferably be decided so as to attain a higher specificity.

In assessment of the possibility of cancer development in a target subject according to this embodiment, the cancer development may include a precancerous condition. The precancerous condition means a general condition involving a significantly increased risk of cancer development. In assessment of the possibility of breast cancer development in a target subject, the breast cancer development may include a breast precancerous condition. In assessment of the possibility of pancreatic cancer development in a target subject, the pancreatic cancer development may include a pancreatic precancerous condition.

<Testing method>

A testing method according to an embodiment of this invention includes steps of: (a) collecting exhaled air from a subject to be assessed (hereinafter, target subject); (b) measuring concentrations of a plurality of types of target components in the exhaled air; and (c) calculating an assessment score from the measurement results of the concentrations, and comparing the assessment score to a certain value or to a certain range of values based on a criterion that cancer is being developed in the subject with a high possibility when the assessment score is equal to the certain value or within the certain range of values.

This testing method is substantially identical to the assessment method, except that the risk of cancer development risk is not determined.

<Assessment device>

An example of the assessment device according to this embodiment is hereinafter described.

An assessment device 1 according to this embodiment includes a measurement unit 3 and a controller 4. The measurement unit 3 is configured to measure concentrations of a plurality of types of target components included in exhaled air collected from a target subject. The controller 4 is configured to calculate an assessment score correlated to the possibility of cancer being developed in the target subject based on measurement results of the concentrations obtained by the measurement unit 3. The controller 4 may be configured to determine a high possibility of cancer being developed in the target subject when the assessment score is equal to a certain value or within a certain range of values. The controller 4 may be configured to calculate the assessment score according to the assessment method or may be further configured to determine the possibility of cancer development.

The assessment device 1 according to this embodiment may provide reliable assessment of the possibility of cancer being developed in a target subject by simply analyzing exhaled air of the target subject without having to perform long-term monitoring.

In the assessment device according to this embodiment, the controller 4 may calculate the assessment score correlated to the possibility of cancer being developed in the target subject irrespective of types of the cancer. The controller 4 may be configured to further determine whether the possibility of cancer being developed in the target subject is high or low based on the assessment score.

As described earlier, the assessment method according to this embodiment may provide reliable assessment of the possibility of breast cancer being developed in the target subject and the possibility of pancreatic cancer being developed in the target subject. In the assessment device according to this embodiment, the controller 4 may calculate an assessment score correlated to the possibility of breast cancer being developed in the target subject. The controller 4 may be configured to determine whether the possibility of breast cancer being developed in the target subject is high or low based on the assessment score. The controller 4 may be configured to calculate an assessment score correlated to the possibility of pancreatic cancer being developed in the target subject or may be further configured to determine whether the possibility of pancreatic cancer being developed in the target subject is high or low based on the assessment score. When the specific type of suspected cancer is identifiable, the target components, assessment score calculating method, and determining method based on the calculated assessment score may preferably be selected and set in accordance with the identified type of cancer.

An example of the assessment device 1 according to this embodiment is hereinafter described in further detail. The assessment device 1 includes, in addition to the measurement unit 3 and the controller 4, a sample inlet 2 and an output unit 5. A carrier gas source 23 is coupled to the assessment device 1.

Exhaled air collected from a target subject is introduced through the sample inlet 2 into the measurement unit 3. The sample inlet 2 communicates with the measurement unit 3 through a flow channel 21. The sample inlet 2 is so structured that the gas component adsorption tube 6 containing exhaled air collected from a test subject is attachable to the sample inlet 2. The gas component adsorption tube 6, being attached to the sample inlet 2, has one opening coupled to the sample inlet 2. The gas component adsorption tube 6, being attached to the sample inlet 2, has another opening coupled to the carrier gas source 23 through a flow channel 22.

The carrier gas source 23 feeds the measurement unit 3 with a carrier gas through the flow channels 21 and 22. Examples of the carrier gas source 23 may include a gas pump or a gas cylinder containing the carrier gas.

In this embodiment, the measurement unit 3 is a gas chromatograph mass spectrometer provided with a separation column 31 and a mass spectrometer 32. Through flow channels, the separation column 31 is coupled to the sample inlet 2, and the mass spectrometer 32 is coupled to the separation column 31. Measurement results of concentrations obtained by the measurement unit 3 are transmitted to the controller 4.

The gas chromatograph mass spectrometer is a nonlimiting example of the measurement unit 3. The measurement unit 3 may instead be a multireactive, real-time mass spectrometer. The multireactive, real-time mass spectrometer may be referred to as a CI ion-selective mass spectrometer. Advantages of the multireactive, real-time mass spectrometer may include high-speed measurement of the concentrations of different types of target components in exhaled air, and high-accuracy measurement of very low concentrations of the target components. The multireactive, real-time mass spectrometer may measure the concentration of any component unmeasurable by the gas chromatograph mass spectrometer, such as water vapor.

An example of the controller 4 is a computer that effectuates functions in accordance with a program run on the computer. The computer includes, as main hardware components, a processor that processes the program and interface devices. Such a computer may be optionally configured, examples of which may include a microcomputer with a memory integral with a processor, and a computer with a memory separate from a processor.

In this embodiment, the program may be recorded on a computer-readable recording medium connected to the computer, or the program may be previously stored in ROM (Read Only Memory) installed in the computer. Another optional example is a computer program product loaded to execute program commands and prompt the computer to effectuate functions of the controller. The program may be written on a recording medium from a program supporting means, or the program may be downloaded through an electrical communication circuit, such as the Internet, and installed in the controller 4.

In this embodiment, the controller 4 is configured to calculate an assessment score correlated to the possibility of cancer being developed in a target subject based on measurement results of the concentrations obtained by the measurement unit 3. The controller 4 may be configured to determine a high possibility of cancer being developed in a target subject when the assessment score is equal to a certain value or within a certain range of values. The controller 4 may be configured to determine a low possibility of cancer being developed in a target subject when the assessment score is not equal to a certain value or beyond a certain range of values. The controller 4 calculates and determines the assessment score in the same manner as described earlier in the step (c) of the assessment method. The controller 4 is further configured to transmit at least one of an assessment result and a determination result to an output destination outside of the controller 4. In this embodiment, the controller 4 transmits at least one of the assessment and determination results to the output unit 5.

The output unit 5 is configured to output at least one of the assessment and the determination results received from the controller 4. The output may be specifically rephrased that information being broadcasted to, for example, an operator of the assessment device 1 in a manner that appeals to any one(s) of the operator's five senses including visual sensation and auditory sensation. The output unit 5 may include, for example, a monitor, a printer, a lamp and combination of two or more thereof.

The operation of the assessment device 1 according to this embodiment is hereinafter described.

With the gas component adsorption tube 6, which contains exhaled air collected from a target subject, being attached to the sample inlet 2, the carrier gas from the carrier gas source 23 flows into the flow channel 22. Then, the exhaled air in the gas component adsorption tube 6 flows with the carrier gas into the flow channel 21 through the sample inlet 2. The exhaled air runs through the flow channel 21 into the separation column 31 of the measurement unit 3. Then, a plurality of different target components included in the exhaled air are separated in the separation column 31. The target components separated in the separation column 31 are then sequentially sent into the mass spectrometer 32. The mass spectrometer 32 generates measurement results of concentrations of the target components, for example, mass spectra. The mass spectrometer 32 transmits the obtained measurement results to the controller 4. The controller 4 calculates an assessment score correlated to the possibility of cancer being developed in the target subject based on the concentration measurement results. Alternatively, the mass spectrometer 32 may determine whether the possibility of cancer being developed in the target subject is high or low. The controller 4 transmits at least one of an assessment result and a determination result to the output unit 5. The output unit 5 outputs at least one of the assessment result and the determination result.

In this embodiment, the assessment device 1 may include a recording means such as a memory. In this instance, the controller 4 may be configured to transmit at least one of the assessment result and the determination result to the recording means and records the transmitted result(s) in the recording means. The assessment device 1 may include a writing means to write a recording result in the recording means. In this instance, the controller 4 may be configured to transmit at least one of the assessment result and the determination result to the writing means and to prompt the writing means to record at least one of the assessment result and the determination result on the recording means. The controller 4 may be configured to transmit at least one of the assessment result and the determination result to an external apparatus outside of the assessment device 1. In this instance, the output unit 5 of the assessment device 1 may be dispensable.

In this embodiment, the sample inlet 2 is so structured that the gas component adsorption tube 6 is attachable thereto. Instead, the sample inlet 2 may be so structured that allows a target subject to directly blow his/her exhaled air into the sample inlet 2.

In this embodiment, the measurement unit 3 is the gas chromatograph mass spectrometer. However, the measurement unit 3 is not particularly limited as far as the measurement unit 3 is an instrument capable of measuring target component concentrations in the exhaled air. For example, the measurement unit 3 may be an infrared spectrophotometer or a sensor gas chromatograph with a gas detection tube, a crystal unit for sample smearing, a separation column, and a semiconductor gas sensor (manufactured by Nissha FIS, Inc.).

The methods for assessing and determining the possibility of cancer development are hereinafter described in further detail and are tabulated in Tables 1 to 8.

<Assessment of possibility of breast cancer development (assessment using threshold)>

In methods hereinafter described, the possibility of breast cancer development is assessed based on thresholds respectively decided for a plurality of types of target components. The methods further determine whether the possibility of breast cancer development is high or low. The concentration of a target component in the methods described below is the ratio of the target component included in exhaled air to the whole exhaled air.

(1) Method 1

A method 1 uses the following target components; 2-methyl-butyric acid, benzaldehyde, and decanal. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 2 points. The possibility of breast cancer development is determined as high when the assessment score is greater than or equal to the reference score. The possibility of breast cancer development is determined as low when the assessment score is less than the reference score.

The possibility of breast cancer being developed in test subjects was assessed and determined by the method 1, in which 7 test subjects among 22 breast cancer subjects were determined as having a high possibility of breast cancer development. The rest of the breast cancer subjects were determined as having a low possibility of breast cancer development. All of 29 non-breast cancer subjects were determined as having a low possibility of breast cancer development. The method 1 resulted in the sensitivity of 31.8% and the specificity of 100%. Thus, the method 1 has an excellent specificity in determining the possibility of breast cancer development.

(2) Method 2

A method 2 uses the following target components; 2-methyl-butyric acid, benzaldehyde, decanal, and phenol. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 2 points. The possibility of breast cancer development is determined as high when the assessment score is less than the reference score. The possibility of breast cancer development is determined as low when the assessment score is less than the reference score.

The possibility of breast cancer being developed in test subjects was assessed and determined by the method 2, in which 15 test subjects among 22 breast cancer subjects were determined as having a high possibility of breast cancer development. The rest of the breast cancer subjects were determined as having a low possibility of breast cancer development. Among 29 non-breast cancer subjects, 28 test subjects were determined as having a low possibility of breast cancer development. One remaining non-breast cancer subject was determined as having a high possibility of breast cancer development. The method 2 resulted in the sensitivity of 68.2% and the specificity of 96.6%. Thus, the method 2 has a high sensitivity and a high specificity in determining the possibility of breast cancer development. Especially, the specificity of this method is notably high.

(3) Method 3

A method 3 uses the following target components; 1-decanol, 3-methyl-1-butanol, 1-hexanol, 1-heptanol, $\alpha,\alpha$-dimethyl-benzene ethanol acetate, 2-methyl-phenol, 2-propenal, nonanal, decanal, benzaldehyde, acetic acid, 2-methyl-butyric acid, ethyl acetate, DL-alanyl-L-alanine, 3,3-dimethyl-(3H)indazole, and 1-methylpyrrolidine. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 7 points. The possibility of breast cancer development is determined as high when the assessment score is greater than or equal to the reference score. The possibility of breast cancer development is determined as low when the assessment score is less than the reference score.

The possibility of breast cancer being developed in test subjects was assessed and determined by the method 3, in which 15 test subjects among 22 breast cancer subjects were determined as having a high possibility of breast cancer development. The rest of the breast cancer subjects were determined as having a low possibility of breast cancer development. All of 29 non-breast cancer subjects were determined as having a low possibility of breast cancer development. The method 3 resulted in the sensitivity of 68.2% and the specificity of 100%. Thus, the method 3 has a high sensitivity and a considerably high specificity in determining the possibility of breast cancer development. Especially, the method achieves an excellently specificity.

(4) Method 4

A method 4 uses the following target components; 3-methyl-1-butanol, nonanal, benzaldehyde, acetic acid, and ethyl acetate. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 4 points. The possibility of breast cancer development is determined as high when the assessment score is greater than or equal to the reference score. The possibility of breast cancer development is determined as low when the assessment score is less than the reference score.

The possibility of breast cancer being developed in test subjects was assessed and determined by the method 4, in which 12 test subjects among 22 breast cancer subjects were determined as having a high possibility of breast cancer development. The rest of the breast cancer subjects were determined as having a low possibility of breast cancer development. Among 29 non-breast cancer subjects, 28 test subjects were determined as having a low possibility of breast cancer development. One remaining non-breast cancer subject was determined as having a high possibility of breast cancer development. The method 4 resulted in the sensitivity of 54.5% and the specificity of 96.6%. Thus, the method 4 has a notably high specificity in determining the possibility of breast cancer development.

(5) Method 5

A method 5 uses the following target components; 1-propanol, 1-octen-3-ol, phenol, heptanal, propionic acid, 2-methyl-propionic acid, butyric acid, acetoin, and mercaptoacetone. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 6 points. The possibility of breast cancer development is determined as high when the assessment score is greater than or equal to the reference score. The possibility of breast cancer development is determined as low when the assessment score is less than the reference score.

The possibility of breast cancer being developed in test subjects was assessed and determined by the method 5, in which 11 test subjects among 25 breast cancer subjects were determined as having a high possibility of breast cancer development. The rest of the breast cancer subjects were determined as having a low possibility of breast cancer development. Among 29 non-breast cancer subjects, 26 test subjects were determined as having a low possibility of breast cancer development. The rest of the non-breast cancer subjects were determined as having a high possibility of breast cancer development. The method 5 resulted in the sensitivity of 44.0% and the specificity of 90.0%. Thus, the method 5 has a notable high specificity in determining the possibility of breast cancer development.

(6) Method 6

A method 6 uses the following target components; acetoin, butyric acid, 1-propanol, mercaptoacetone, 1-methylpyrrolidine, and 1-octen-3-ol. The method 6 includes a first assessment and a second assessment. A determination result based on the first assessment takes precedence over the other.

The target components used for the first assessment are acetoin, butyric acid, and 1-propanol. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 2 points. The possibility of breast cancer development is determined as high when the assessment score is greater than or equal to the reference score. When the assessment score is less than the reference score, this method, without determining the possibility at this stage, proceeds to the second assessment.

The target components used for the second assessment are mercaptoacetone, 1-methylpyrrolidine, and 1-octen-3-ol. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 2 points. The possibility of breast cancer development is determined as high when the assessment score is greater than or equal to the reference score. The possibility of breast cancer development is determined as low when the assessment score is less than the reference score.

The possibility of breast cancer being developed in test subjects was assessed and determined by the method 6. Among 25 breast cancer subjects, 21 test subjects were determined as having a high possibility of breast cancer development. The rest of the breast cancer subjects were determined as having a low possibility of breast cancer development. Among 29 non-breast cancer subjects, 18 test subjects were determined as having a low possibility of breast cancer development. The rest of the non-breast cancer subjects were determined as having a high possibility of breast cancer development. The method 6 resulted in the sensitivity of 84.0% and the specificity of 62.1%. Thus, the method 6 has a high sensitivity and a high specificity in determining the possibility of breast cancer development. Especially, the sensitivity of this method is notably high.

(7) Method 7

A method 7 uses the following target components; butyric acid, phenol, trimethylamine, acetoin, mercaptoacetone, and 1-octen-3-ol. These target components are divided into a first group consisting of butyric acid, phenol, trimethylamine, and acetoin, and a second group consisting of mercaptoacetone and 1-octen-3-ol. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The score of the first group is the points in total acquired by the target components included in the first group. The score of the second group is 1 point when the points in total acquired by the target components of this group are greater than or equal to 1 point, and is 0 point when the points in total are 0 point. The assessment score is the scores in total of the first and second groups, and the reference score is 2 points. The possibility of breast cancer development is determined as high when the assessment score is greater than or equal to the reference score. The possibility of breast cancer development is determined as low when the assessment score is less than the reference score.

The possibility of breast cancer development was assessed and determined by the method 7 in 39 breast cancer subjects and 56 non-breast cancer subjects. This method resulted in the sensitivity of 77% and the specificity of 55%. Thus, the method 7 has a high sensitivity in determining the possibility of breast cancer development.

(8) Method 8

A method 8 uses the following target components; 2-methyl-propionic acid, acetic acid, butyric acid, propionic acid, phenol, mercaptoacetone, and 1-octen-3-ol. The method 8 includes a first assessment and a second assessment. A determination result based on the first assessment takes precedence over the other.

The target components used for the first assessment are 2-methyl-propionic acid, acetic acid, butyric acid, and propionic acid. The threshold of each target component is a median of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 1 point. When the assessment score is greater than or equal to the reference score, this method, without determining the possibility at this stage, proceeds to the second assessment. The possibility of breast cancer development is determined as low when the assessment score is less than the reference score.

The target components used for the second assessment are mercaptoacetone and 1-octen-3-ol. The threshold of each target component is a median of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 1 point. The possibility of breast cancer development is determined as high when the assessment score is greater than or equal to the reference score. The possibility of breast cancer development is determined as low when the assessment score is less than the reference score.

The possibility of breast cancer development was assessed and determined by the method 8 in 39 breast cancer subjects and 56 non-breast cancer subjects. This method resulted in the sensitivity of 77% and the specificity of 42%. Thus, the method 8 has a high sensitivity in determining the possibility of breast cancer development.

(9) Method 9

A method 9 uses the following target components; 2-methyl-propionic acid, acetic acid, butyric acid, propionic acid, trimethylamine, 2-methylfuran, 1-propanol, butanone, and acetoin. The method 9 includes a first assessment and a second assessment. A determination result based on the first assessment takes precedence over the other.

The target components used for the first assessment are 2-methyl-propionic acid, acetic acid, butyric acid, and propionic acid. The threshold of each target component is a median of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 1 point. When the assessment score is greater than or equal to the reference score, this method, without determining the possibility at this stage, proceeds to the second assessment. The possibility of breast cancer development is determined as low when the assessment score is less than the reference score.

The target components used for the second assessment are trimethylamine, 2-methylfuran, 1-propanol, butanone, and acetoin. The threshold of each target component is a median of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 2 points. The possibility of breast cancer development is determined as high when the assessment score is greater than or equal to the reference score. The possibility of breast cancer development is determined as low when the assessment score is less than the reference score.

The possibility of breast cancer development was assessed and determined by the method 9 in 39 breast cancer subjects and 56 non-breast cancer subjects. This method resulted in the sensitivity of 93% and the specificity of 13%. Thus, the method 9 has a particularly high specificity in determining the possibility of breast cancer development.

(10) Method 10

A method 10 uses the following target components; 2-methyl-propionic acid, acetic acid, butyric acid, propionic acid, phenol, and benzaldehyde. The target components are divided into a first group consisting of 2-methyl-propionic acid, acetic acid, butyric acid, and propionic acid, and a second group consisting of phenol and benzaldehyde. The threshold of each target component is a median of concentrations of the target component in exhaled air of non-breast cancer subjects. Each target component acquires 1 point when its concentration in exhaled air is greater than or equal to the threshold, and 0 point when the concentration is less than the threshold. The score of the first group is 1 point when the points in total acquired by the target components of this group are greater than or equal to 1 point, and is 0 point when the points in total are 0 point. The score of the second group is the points in total acquired by the target components included in the second group. The assessment score is the scores in total of the first and second groups, and the reference score is 1 point. The possibility of breast cancer development is determined as high when the assessment score is greater than or equal to the reference score. The possibility of breast cancer development is determined as low when the assessment score is less than the reference score.

The possibility of breast cancer development was assessed and determined by the method 10 in 39 breast cancer subjects and 56 non-breast cancer subjects. This method resulted in the sensitivity of 93% and the specificity of 13%. Thus, the method 10 has a markedly high sensitivity in determining the possibility of breast cancer development.

(11) Method 11

A method 11 uses the following target components; 2-methyl-propionic acid, acetic acid, butyric acid, propionic acid, phenol, and benzaldehyde. The target components are divided into a first group consisting of 2-methyl-propionic acid, acetic acid, butyric acid, and propionic acid, and a second group consisting of phenol and benzaldehyde. The threshold of each target component is a median of concentrations of the target component in exhaled air of non-breast cancer subjects. Each target component acquires 1 point when its concentration in exhaled air is greater than or equal to the threshold, and 0 point when the concentration is less than the threshold. The score of the first group is 1 point when the points in total acquired by the target components of this group are greater than or equal to 2 points, and is 0 point when the points in total are less than or equal to 1 point. The score of the second group is the points in total acquired by the target components included in the second group.

The assessment score is the scores in total of the first and second groups, and the reference score is 2 points. The possibility of breast cancer development is determined as high when the assessment score is greater than or equal to the reference score. The possibility of breast cancer development is determined as low when the assessment score is less than the reference score.

The possibility of breast cancer development was assessed and determined by the method 11 in 39 breast cancer subjects and 56 non-breast cancer subjects. This method resulted in the sensitivity of 87% and the specificity of 23%. Thus, the method 11 has a very high sensitivity in determining the possibility of breast cancer development.

(12) Method 12

A method 12 uses the following target components; 2-methyl-propionic acid, acetic acid, butyric acid, propionic acid, phenol, and benzaldehyde. The target components are divided into a first group consisting of 2-methyl-propionic acid, acetic acid, butyric acid, and propionic acid, and a second group consisting of phenol and benzaldehyde. The threshold of each target component includes a first threshold and a second threshold. The first threshold of each target component is a median of concentrations of the target component in exhaled air of non-breast cancer subjects. The second threshold of each target component is a third quartile of concentrations of the target component in exhaled air of non-breast cancer subjects. Each target component acquires 2 points when its concentration in exhaled air is greater than or equal to the second threshold, 1 point when the concentration is greater than or equal to the first threshold and less than the second threshold, and 0 point when the concentration is less than the first threshold. The score of the first group is 1 point when the points in total acquired by the target components of this group are greater than or equal to 2 points, and is 0 point when the points in total are less than or equal to 1 point. The score of the second group is the points in total acquired by the target components included in the second group. The assessment score is the scores in total of the first and second groups, and the reference score is 3 points. The possibility of breast cancer development is determined as high when the assessment score is greater than or equal to the reference score. The possibility of breast cancer development is determined as low when the assessment score is less than the reference score.

The possibility of breast cancer development was assessed and determined by the method 12 in 39 breast cancer subjects and 56 non-breast cancer subjects. This method resulted in the sensitivity of 70% and the specificity of 58%. Thus, the method 12 has a high sensitivity in determining the possibility of breast cancer development.

(13) Method 13

A method 13 uses the following target components; 2-methyl-propionic acid, acetic acid, butyric acid, propionic acid, phenol, and benzaldehyde. The target components are divided into a first group consisting of 2-methyl-propionic acid, acetic acid, butyric acid, and propionic acid, and a second group consisting of phenol and benzaldehyde. The threshold of each target component includes a first threshold and a second threshold. The first threshold of each target component is a median of concentrations of the target component in exhaled air of non-breast cancer subjects. The second threshold of each target component is a third quartile of concentrations of the target component in exhaled air of non-breast cancer subjects. Each target component acquires 2 points when its concentration in exhaled air is greater than or equal to the second threshold, 1 point when the concentration is greater than or equal to the first threshold and less than the second threshold, and 0 point when the concentration is less than the first threshold. The score of the first group is 1 point when the points in total acquired by the target components of this group are greater than or equal to 4 points, and is 0 point when the points in total are less than or equal to 3 points. The score of the second group is the points in total acquired by the target components included in the second group. The assessment score is the scores in total of the first and second groups, and the reference score is 4 points. The possibility of breast cancer development is determined as high when the assessment score is greater than or equal to the reference score. The possibility of breast cancer development is determined as low when the assessment score is less than the reference score.

The possibility of breast cancer development was assessed and determined by the method 13 in 39 breast cancer subjects and 56 non-breast cancer subjects. This method resulted in the sensitivity of 60% and the specificity of 65%. Thus, the method 13 has a high sensitivity and a high specificity in determining the possibility of breast cancer development.

<Assessment of possibility of pancreatic cancer development (assessment using threshold)>

In methods hereinafter described, the possibility of pancreatic cancer development is assessed based on thresholds respectively decided for a plurality of types of target components. The methods further determine whether the possibility of pancreatic cancer development is high or low. The concentration of a target component in the methods described below is the ratio of the target component included in exhaled air to the whole exhaled air.

(14) Method 14

A method 14 uses the following target components; 1-propanol, 1,2-ethanediol, phenol, propionic acid, 2-methyl-propionic acid, butyric acid, heptanal, decanal, butanal, 2,3-butanedione, and acetoin. The target components are divided into a first group consisting of 1-propanol, 1,2-ethanediol, and phenol, a second group consisting of propionic acid, 2-methyl-propionic acid, and butyric acid, a third group consisting of heptanal, decanal, and butanal, and a fourth group consisting of 2,3-butanedione and acetoin. The threshold of each target component is a third quartile of concentrations of the target component in exhaled air of non-breast cancer subjects. The score of the first group is 1 point when the points in total acquired by the target components of this group are greater than or equal to 2 points, and is 0 point when the points in total are less than or equal to 1 point. The score of the second group is 1 point when the points in total acquired by the target components of this group are greater than or equal to 2 points, and is 0 point when the points in total are less than or equal to 1 point. The score of the third group is 1 point when the points in total acquired by the target components of this group are greater than or equal to 1 point, and is 0 point when the points in total are 0 point. The score of the fourth group is 1 point when the points in total acquired by the target components of this group are greater than or equal to 1 point, and is 0 point when the points in total are 0 point. The assessment score is the scores in total of the first, second, third, and fourth groups, and the reference score is 3 points. The possibility of pancreatic cancer development is determined as high when the assessment score is greater than or equal to the reference score. The possibility of breast cancer development is determined as low when the assessment score is less than the reference score.

The possibility of pancreatic cancer being developed in test subjects was assessed and determined by the method 14. Among 31 pancreatic cancer subjects, 17 test subjects were determined as having a high possibility of pancreatic cancer development. The rest of the pancreatic cancer subjects were determined as having a low possibility of pancreatic cancer development. Among 58 non-pancreatic cancer subjects, 57 test subjects were determined as having a low possibility of pancreatic cancer development. The rest of the non-pancreatic cancer subjects were determined as having a high possibility of pancreatic cancer development. The method 14 resulted in the sensitivity of 55% and the specificity of 98%. Thus, the method 14 has a relatively high sensitivity and a high specificity in determining the possibility of pancreatic cancer development. Especially, the specificity of this method is notably high.

(15) Method 15

The target components used by a method 15 are one selected from 1-propanol and phenol, one selected from heptanal and decanal, one selected from the group consisting of propionic acid, 2-methyl-propionic acid, and butyric acid, and one selected from the group consisting of 2,3-butanedione and acetoin. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The reference score is 3 points. The possibility of pancreatic cancer development is determined as high when the assessment score is greater than or equal to the reference score. The possibility of pancreatic cancer development is determined as low when the assessment score is less than the reference score.

The possibility of pancreatic cancer being developed in test subjects was assessed and determined by the method 15. This method successfully determined the possibility of pancreatic cancer development in the test subjects including 31 pancreatic cancer subjects and 58 non-pancreatic cancer subjects by the sensitivity of 19 to 32% and the specificity of 91 to 100%. Thus, the method 15 has a considerably high specificity in determining the possibility of pancreatic cancer development.

The specificity of this method in determining the possibility of pancreatic cancer development was particularly high when the target components were 1-propanol, decanal, propionic acid, and 2,3-butanedione, when the target components were 1-propanol, decanal, 2-methyl-propionic acid, and 2,3-butanedione, when the target components were phenol, heptanal, 2-methyl-propionic acid, and 2,3-butanedione, and when the target components were phenol, decanal, 2-methyl-propionic acid, and 2,3-butanedione.

(16) Method 16

The target components used by a method 16 are phenol, butyric acid, acetoin, one selected from heptanal and decanal, and one selected from the group consisting of carbon disulfide, 1-octen-3-ol, benzaldehyde, acetaldehyde, acetic acid, and 2-methylfuran. The target components may include neither heptanal nor decanal. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 3 points. The possibility of pancreatic cancer development is determined as high when the assessment score is greater than or equal to the reference score. The possibility of pancreatic cancer development is determined as low when the assessment score is less than the reference score.

The possibility of pancreatic cancer being developed in test subjects was assessed and determined by the method 16. This method successfully determined the possibility of pancreatic cancer development in the test subjects including 31 pancreatic cancer subjects and 58 non-pancreatic cancer subjects by the sensitivity of 39 to 58% and the specificity of 84 to 100%. Thus, the method 16 has a considerably high specificity in determining the possibility of pancreatic cancer development.

The specificity of this method in determining the possibility of pancreatic cancer development was particularly high when the target components were phenol, butyric acid, acetoin, decanal, and carbon disulfide, when the target components were phenol, butyric acid, acetoin, decanal, and benzaldehyde, when the target components were phenol, butyric acid, acetoin, decanal, and 2-methylfuran, and when the target components were phenol, butyric acid, acetoin, heptanal, and 2-methylfuran.

(17) Method 17

The target components used by a method 17 are phenol, decanal, 2,3-butanedione, one selected from propionic acid and 2-methyl-propionic acid, and one selected from the group consisting of carbon disulfide, 1-octen-3-ol, benzaldehyde, acetaldehyde, acetic acid, and 2-methylfuran. The target components may include neither of propionic acid nor 2-methyl-propionic acid. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 3 points. The possibility of pancreatic cancer development is determined as high when the assessment score is greater than or equal to the reference score. The possibility of pancreatic cancer development is determined as low when the assessment score is less than the reference score.

The possibility of pancreatic cancer being developed in test subjects was assessed and determined by the method 17. This method successfully determined the possibility of pancreatic cancer development in the test subjects including 31 pancreatic cancer subjects and 58 non-pancreatic cancer subjects by the sensitivity of 16 to 48% and the specificity of 86 to 98%. Thus, the method 17 has a particularly high specificity in determining the possibility of pancreatic cancer development.

The specificity of this method in determining the possibility of pancreatic cancer development was particularly high when the target components were phenol, decanal, 2,3-butanedione, and carbon disulfide, when the target components were phenol, decanal, 2,3-butanedione, and 2-methylfuran, and when the target components were phenol, decanal, 2,3-butanedione, propionic acid, and carbon disulfide, and when the target components were phenol, decanal, 2,3-butanedione, propionic acid, and 1-octen-3-ol.

(18) Method 18

The target components used by a method 18 are 1-methylpyrrolidine, one selected from 2-propanol and 1-octen-3-ol, one selected from heptanal and decanal, one selected from the group consisting of propionic acid, 2-methyl-propionic acid, and butyric acid, and one selected from butanone and acetoin. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 4 points. The possibility of pancreatic cancer development is determined as high when the assessment score is greater than or equal to the reference score. The possibility of pancreatic cancer development is determined as low when the assessment score is less than the reference score.

The possibility of pancreatic cancer being developed in test subjects was assessed and determined by the method 18. This method successfully determined the possibility of pancreatic cancer development in the test subjects including 31 pancreatic cancer subjects and 58 non-pancreatic cancer subjects by the sensitivity of 26 to 39% and the specificity of 93 to 96%. Thus, the method 18 has a markedly high specificity in determining the possibility of pancreatic cancer development.

The specificity of this method in determining the possibility of pancreatic cancer development was particularly high when the target components were 1-methylpyrrolidine, 2-propanol, decanal, butyric acid, and acetoin, when the target components were 1-methylpyrrolidine, 2-propanol, decanal, propionic acid, and butanone, when the target components were 1-methylpyrrolidine, 2-propanol, heptanal, butyric acid, and butanone, and when the target components were 1-methylpyrrolidine, 2-propanol, heptanal, propionic acid, and butanone.

(19) Method 19

The target components used by a method 19 are 1-methylpyrrolidine, phenol, one selected from heptanal and decanal, one selected from the group consisting of propionic acid, 2-methyl-propionic acid, and butyric acid, and one selected from butanone and acetoin. The threshold of each target component is a third quartile of concentrations of the target component in exhaled air of non-breast cancer subjects. Each target component acquires 1 point when its concentration in exhaled air is greater than or equal to the threshold, and 0 point when the concentration is less than the threshold. The assessment score is the points in total of the target components, and the reference score is 4 points. One type of target component is selected from each of first to fifth assessments. The possibility of pancreatic cancer development is determined as high when the assessment scores in total of these assessments are greater than or equal to the reference score. The possibility of pancreatic cancer development is determined as low when the assessment scores in total are less than the reference score.

The possibility of pancreatic cancer being developed in test subjects was assessed and determined by the method 19. This method successfully determined the possibility of pancreatic cancer development in the test subjects including 31 pancreatic cancer subjects and 58 non-pancreatic cancer subjects by the sensitivity of 19 to 32% and the specificity of 91 to 100%. Thus, the method 19 has a considerably high specificity in determining the possibility of pancreatic cancer development.

The specificity of this method in determining the possibility of pancreatic cancer development was particularly high when the target components were 1-methylpyrrolidine, phenol, decanal, propionic acid, and acetoin, when the target components were 1-methylpyrrolidine, phenol, heptanal, 2-methyl-propionic acid, and butanone, when the target components were 1-methylpyrrolidine, phenol, heptanal, butyric acid, and acetoin, and when the target components were 1-methylpyrrolidine, phenol, decanal, propionic acid, and butanone.

(20) Method 20

A method 20 uses the following target components; trimethylamine, decanal, acetophenone, acetic acid, mercaptoacetone, 2-methyl-propionic acid, 1-methylpyrrolidine, and phenol. These target components are divided into a first group consisting of trimethylamine, decanal, acetophenone, acetic acid, and mercaptoacetone, and a second group consisting of 2-methyl-propionic acid, 1-methylpyrrolidine, and phenol. The threshold of each target component is a third quartile of concentrations of the target component in exhaled air of non-pancreatic cancer subjects. Each target component acquires 1 point when its concentration in exhaled air is greater than or equal to the threshold, and 0 point when the concentration is less than the threshold. The score of the first group is the points in total acquired by the target components included in the first group. The score of the second group is 1 point when the points in total acquired by the target components of this group are greater than or equal to 1 point, and is 0 point when the points in total are 0 point. The assessment score is the scores in total of the first and second groups, and the reference score is 3 points. The possibility of pancreatic cancer development is determined as high when the assessment scores in total of first and second assessments are greater than or equal to the reference score. The possibility of pancreatic cancer development is determined as low when the assessment scores in total are less than the reference score.

The possibility of pancreatic cancer being developed in test subjects; 34 pancreatic cancer subjects and 45 non-pancreatic cancer subjects, was assessed and determined by the method 20, which resulted in the sensitivity of 67% and the specificity of 89%.

Thus, the method 20 has a very high specificity in determining the possibility of pancreatic cancer development.

(21) Method 21

A method 21 uses the following target components; trimethylamine, decanal, acetophenone, acetic acid, mercaptoacetone, 2-methyl-propionic acid, 1-methylpyrrolidine, and phenol. These target components are divided into a first group consisting of trimethylamine, decanal, acetophenone, acetic acid, and mercaptoacetone, and a second group consisting of 2-methyl-propionic acid, 1-methylpyrrolidine, and phenol. In this method, concentrations of each target component in exhaled air collected from non-pancreatic cancer subjects are divided by concentrations of carbon disulfide in the exhaled air, and a median of the values thus obtained is used as the threshold of each target component. To decide the score of each target component from its concentration, concentrations of the target component are divided by concentrations of carbon disulfide in exhaled air to calculate a reduced value. Each target component acquires 1 point when its reduced value is greater than or equal to the threshold, and 0 point when the concentration is less than the threshold. The score of the first group is the points in total acquired by the target components included in the first group. The score of the second group is 1 point when the points in total acquired by the target components of this group are greater than or equal to 1 point, and is 0 point when the points in total are 0 point. The assessment score is the scores in total of the first and second groups, and the reference score is 4 points. The possibility of pancreatic cancer development is determined as high when the assessment score is greater than or equal to the reference score. The possibility of pancreatic cancer development is determined as low when the assessment scores in total are less than the reference score.

The possibility of pancreatic cancer being developed in test subjects; 34 pancreatic cancer subjects and 45 non-pancreatic cancer subjects, was assessed and determined by the method 21, which resulted in the sensitivity of 67% and the specificity of 83%. Thus, the method 21 has a very high specificity in determining the possibility of pancreatic cancer development.

This method 21, when deciding the score of each target component from its concentration, uses a reduced value calculated by division of concentrations of the target component by concentrations of carbon disulfide in exhaled air. Then, concentrations of each target component may be standardized by carbon disulfide considered to correlate to amounts of exhaled air collected from test subjects.

(22) Method 22

A method 22 uses the following target components; trimethylamine, acetophenone, 1-methylpyrrolidine, mercaptoacetone, 2-methylfuran, phenol, 1-propanol, acetoin, propionic acid, 2-methyl-propionic acid, butyric acid, and acetic acid. These target components are divided into a first group consisting of trimethylamine, acetophenone, 1-methylpyrrolidine, and mercaptoacetone, a second group consisting of 2-methylfuran, phenol, 1-propanol, and acetoin, and a third group consisting of propionic acid, 2-methyl-propionic acid, butyric acid, and acetic acid. The threshold of each target component is a median of concentrations of the target component in exhaled air of test subjects (pancreatic cancer subjects and non-pancreatic cancer subjects). Each target component acquires 1 point when its concentration in exhaled air is greater than or equal to the threshold, and 0 point when the concentration is less than the threshold. The score of the first group is the points in total acquired by the target components included in the first group. When the points in total of the target components included in the third group are 0 point, the score of the second group is unexceptionally 0 point regardless of any point acquired by the target components included in this group. Provided that the points in total of the target components included in the third group are greater than or equal to 1 point, the score of the second group is 1 point when the points in total acquired by the target components included in the second group are greater than or equal to 1 point but is 0 point when the points in total acquired by the target components included in the second group are 0 point. The assessment score is the scores in total of the first and second groups, and the reference score is 3 points. The possibility of pancreatic cancer development is determined as high when the scores in total of the assessments are greater than or equal to the reference score, and is determined as low when the scores in total of the respective assessments are less than the reference score.

The possibility of pancreatic cancer development was assessed and determined by the method 22 in 34 pancreatic cancer subjects and 45 non-pancreatic cancer subjects. This method resulted in the sensitivity of 27% and the specificity of 100%. Thus, the method 22 has an excellent specificity in determining the possibility of pancreatic cancer development.

Thus, the method 22 may achieve marked improvement in specificity by the rule change, i.e., decision of the score of the second group depends on the score of the third group.

(23) Method 23

A method 23 uses the following target components; propionic acid, 2-methyl-propionic acid, butyric acid, acetic acid, acetophenone, and benzaldehyde. The method 23 includes a first assessment and a second assessment. A determination result based on the first assessment takes precedence over the other.

The target components used for the first assessment are propionic acid, 2-methyl-propionic acid, butyric acid, and acetic acid. The threshold of each target component is a median of concentrations of the target component in exhaled air of test subjects (pancreatic cancer subjects and non-pancreatic cancer subjects). Each target component acquires 1 point when its concentration in exhaled air is greater than or equal to the threshold, and 0 point when the concentration is less than the threshold. The assessment score is the points in total of the target components, and the reference score is 1 point. When the assessment score is greater than or equal to the reference score, this method, without determining the possibility at this stage, proceeds to the second assessment. The possibility of pancreatic cancer development is determined as low when the assessment scores in total are less than the reference score.

The target components used for the second assessment are acetophenone and benzaldehyde. The threshold of each target component is a median of concentrations of the target component in exhaled air of test subjects (pancreatic cancer subjects and non-pancreatic cancer subjects). Each target component acquires 1 point when its concentration in exhaled air is greater than or equal to the threshold, and 0 point when the concentration is less than the threshold. The assessment score is the points in total of the target components, and the reference score is 1 point. The possibility of pancreatic cancer development is determined as high when the assessment score is greater than or equal to the reference score. The possibility of pancreatic cancer development is determined as low when the assessment scores in total are less than the reference score.

The possibility of pancreatic cancer being developed in test subjects; 34 pancreatic cancer subjects and 45 non-pancreatic cancer subjects, was assessed and determined by the method 23, which resulted in the sensitivity of 52% and the specificity of 89%. Thus, the method 23 has a very high specificity in determining the possibility of pancreatic cancer development.

(24) Method 24

A method 24 uses the following target components; propionic acid, 2-methyl-propionic acid, butyric acid, acetic acid, acetophenone, and benzaldehyde. The target components are divided into a first group consisting of propionic acid, 2-methyl-propionic acid, butyric acid, and acetic acid, and a second group consisting of acetophenone and benzaldehyde. The threshold of each target component is a median of concentrations of the target component in exhaled air of test subjects (pancreatic cancer subjects and non-pancreatic cancer subjects). Each target component acquires 1 point when its concentration in exhaled air is greater than or equal to the threshold, and 0 point when the concentration is less than the threshold. The score of the first group is the points in total acquired by the target components included in the first group. The score of the second group is 1 point when the points in total acquired by the target components of this group are greater than or equal to 1 point, and is 0 point when the points in total are 0 point. The assessment score is the scores in total of the first and second groups, and the reference score is 3 points. The possibility of pancreatic cancer development is determined as high when the assessment score is greater than or equal to the reference score. The possibility of pancreatic cancer development is determined as low when the assessment scores in total are less than the reference score.

The possibility of pancreatic cancer being developed in test subjects was assessed and determined by the method 24, which resulted in the sensitivity of 67% and the specificity of 86%. Thus, the method 24 has a very high specificity in determining the possibility of pancreatic cancer development.

(25) Method 25

A method 25 uses the following target components; propionic acid, 2-methyl-propionic acid, butyric acid, acetic acid, acetophenone, benzaldehyde, and trimethylamine. The target components are divided into a first group consisting of propionic acid, 2-methyl-propionic acid, butyric acid, and acetic acid, and a second group consisting of acetophenone, benzaldehyde, and trimethylamine. The threshold of each target component, except for benzaldehyde and trimethylamine, is a median of concentrations of the target component in exhaled air of test subjects (pancreatic cancer subjects and non-pancreatic cancer subjects). The threshold of benzaldehyde, trimethylamine is a third quartile of its concentration in exhaled air of test subjects (pancreatic cancer subjects and non-pancreatic cancer subjects). Each target component acquires 1 point when its concentration in exhaled air is greater than or equal to the threshold, and 0 point when the concentration is less than the threshold. The score of the first group is 1 point when the points in total acquired by the target components of this group are greater than or equal to 1 point, and is 0 point when the points in total are 0 point. The score of the second group is the points in total acquired by the target components included in the second group. The assessment score is the scores in total of the first and second groups, and the reference score is 3 points. The possibility of pancreatic cancer development is determined as high when the assessment scores in total of first and second assessments are greater than or equal to the reference score. The possibility of pancreatic cancer development is determined as low when the assessment scores in total are less than the reference score.

The possibility of pancreatic cancer being developed in test subjects was assessed and determined by the method 25, which resulted in the sensitivity of 67% and the specificity of 89%. Thus, the method 25 has a very high specificity in determining the possibility of pancreatic cancer development.

(26) Method 26

A method 26 uses the following target components; acetophenone, benzaldehyde, and phenol. The threshold of each target component is a median of concentrations of the target component in exhaled air of test subjects (pancreatic cancer subjects and non-pancreatic cancer subjects). Each target component acquires 1 point when its concentration in exhaled air is greater than or equal to the threshold, and 0 point when the concentration is less than the threshold. The assessment score is the points in total of the target components, and the reference score is 2 points. The possibility of pancreatic cancer development is determined as high when the assessment score is greater than or equal to the reference score. The possibility of pancreatic cancer development is determined as low when the assessment score is less than the reference score.

The possibility of pancreatic cancer being developed in test subjects was assessed and determined by the method 26, which resulted in the sensitivity of 73% and the specificity of 71%. Thus, the method 26 has a high sensitivity and a high specificity in determining the possibility of pancreatic cancer development.

(27) Method 27

A method 27 uses the following target components; decanal, and butyric acid. The threshold of each target component is a third quartile of concentrations of the target component in exhaled air of test subjects. Each target component acquires 1 point when its concentration in exhaled air is greater than or equal to the threshold, and 0 point when the concentration is less than the threshold. The assessment score is the points in total of the target components, and the reference score is 2 points.The possibility of pancreatic cancer development is determined as high when the assessment score is equal to the reference score. The possibility of pancreatic cancer development is determined as low when the assessment scores in total are less than the reference score.

The possibility of pancreatic cancer development was assessed and determined by the method 27 in 34 pancreatic cancer subjects, 18 diseased subjects, and 27 subjects affected with neither of pancreatic cancer nor any other disease. This method resulted in the sensitivity of 44% and the specificity of 93%. Thus, the method 27 has a notably high specificity in determining the possibility of pancreatic cancer development.

The possibility of pancreatic cancer development was assessed and determined by the method 27 in, among all of test subjects, pancreatic cancer subjects and non-pancreatic cancer subjects who were affected with pancreatitis, polyp of large intestine, or adhesive intestinal obstruction (hereinafter, may be collectively referred to as diseased subjects). This method resulted in the specificity of 83%. Thus, the method 27 has a very high specificity in determining the possibility of pancreatic cancer being developed in target subjects including subjects affected with any disease but pancreatic cancer.

(28) Method 28

A method 28 uses the following target components; decanal, butyric acid, and 2-methyl-propionic acid. The threshold of each target component is a third quartile of concentrations of the target component in exhaled air of test subjects. Each target component acquires 1 point when its concentration in exhaled air is greater than or equal to the threshold, and 0 point when the concentration is less than the threshold. The assessment score is the points in total of the target components, and the reference score is 3 points. The possibility of pancreatic cancer development is determined as high when the assessment score is equal to the reference score. The possibility of pancreatic cancer development is determined as low when the assessment score is less than the reference score.

The possibility of pancreatic cancer development was assessed and determined by the method 28 in 34 pancreatic cancer subjects, 18 diseased subjects, and 27 subjects affected with neither of pancreatic cancer nor any other disease. This method resulted in the sensitivity of 29% and the specificity of 96%. Thus, the method 28 has a particularly high specificity in determining the possibility of pancreatic cancer development.

The possibility of pancreatic cancer development was assessed and determined by the method 28 in, among all of test subjects, pancreatic cancer subjects and diseased subjects, which resulted in the specificity of 89%. Thus, the method 28 has a very high specificity in determining the possibility of pancreatic cancer being developed in target subjects including subjects affected with any disease but pancreatic cancer.

(29) Method 29

A method 29 uses the following target components; decanal, butyric acid, and trimethylamine. The threshold of each target component is a third quartile of concentrations of the target component in exhaled air of test subjects. Each target component acquires 1 point when its concentration in exhaled air is greater than or equal to the threshold, and 0 point when the concentration is less than the threshold. The assessment score is the points in total of the target components, and the reference score is 3 points. The possibility of pancreatic cancer development is determined as high when the assessment score is equal to the reference score. The possibility of pancreatic cancer development is determined as low when the assessment scores in total are less than the reference score.

The possibility of pancreatic cancer development was assessed and determined by the method 29 in 34 pancreatic cancer subjects, 18 diseased subjects, and 27 subjects affected with neither of pancreatic cancer nor any other disease. This method resulted in the sensitivity of 18% and the specificity of 100%. Thus, the method 29 has an excellent specificity in determining the possibility of pancreatic cancer development.

The possibility of pancreatic cancer development was assessed and determined by the method 29 in, among all of test subjects, pancreatic cancer subjects and diseased subjects, which resulted in the specificity of 100%. Thus, the method 30 has an excellent specificity in determining the possibility of pancreatic cancer being developed in target subjects including subjects affected with any disease but pancreatic cancer.

(30) Method 30

A method 30 uses the following target components; 1-octen-3-ol, 2-methylfuran, decanal, butyric acid, and trimethylamine. The threshold of each target component is a third quartile of concentrations of the target component in exhaled air of test subjects. Each target component acquires 1 point when its concentration in exhaled air is greater than or equal to the threshold, and 0 point when the concentration is less than the threshold. The assessment score is the points in total of the target components, and the reference score is 3 points. The possibility of pancreatic cancer development is determined as high when the assessment score is greater than or equal to the reference score. The possibility of pancreatic cancer development is determined as low when the assessment scores in total are less than the reference score.

The possibility of pancreatic cancer development was assessed and determined by the method 30 in 34 pancreatic cancer subjects, 18 diseased subjects, and 27 subjects affected with neither of pancreatic cancer nor any other disease. This method resulted in the sensitivity of 44% and the specificity of 96%. Thus, the method 30 has a particularly high specificity in determining the possibility of pancreatic cancer development.

The possibility of pancreatic cancer development was assessed and determined by the method 30 in, among all of test subjects, pancreatic cancer subjects and diseased subjects, which resulted in the specificity of 94%. Thus, the method 30 has an excellent specificity in determining the possibility of pancreatic cancer being developed in target subjects including subjects affected with any disease but pancreatic cancer.

<Assessment of possibility of breast cancer development (assessment using standardized values and their levels of contribution)>

In a method hereinafter described, the possibility of breast cancer development is assessed based on standardized values of a plurality of types of target components and their levels of contribution. The methods further determine whether the possibility of breast cancer development is high or low. The concentration of a target component in the method described below is the ratio of the target component included in exhaled air to the whole exhaled air.

(31) Method 31

A method 31 uses the following target components; 1,2-ethanediol, decanal, mercaptoacetone, heptanal, 6-methyl-5-hepten-2-one, cyclohexane, benzaldehyde, 1-pentanol, decane, 2,2,4-trimethyl-hexane, dodecane, 2-methyl-butyric acid, 1-butanol, n-hexane, 1-octen-3-ol, 4-ethylcyclohexanol, carbon disulfide, 2,4-bis(1,1-dimethylethyl)-phenol, nitrosomethylurethane, acetoin. The assessment score is calculated by Formula (1). The standardized value calculating method, contribution deciding method, and reference score deciding method using Formula (1) are characterized as described earlier. The possibility of breast cancer development is determined as high when the assessment score is greater than or equal to the reference score. The possibility of breast cancer development is determined as low when the assessment score is less than the reference score.

The possibility of breast cancer development was assessed and determined by the method 31 in 39 breast cancer subjects and 56 non-breast cancer subjects. Then, all of the breast cancer subjects were determined as having a high possibility of breast cancer development, and all of the non-breast cancer subjects were determined as having a low possibility of breast cancer development. The method 31 resulted in the sensitivity of 79% and the specificity of 93%. Thus, the method 31 has a very high sensitivity and a markedly high specificity in determining the possibility of breast cancer development.

<Assessment of possibility of pancreatic cancer development (assessment using standardized values and their levels of contribution)>

In a method hereinafter described, the possibility of pancreatic cancer development is assessed based on standardized values of a plurality of types of target components and their levels of contribution. The method further determines whether the possibility of pancreatic cancer development is high or low. The concentration of a target component in the method described below is the ratio of the target component included in exhaled air to the whole exhaled air.

(32) Method 32

A method 32 uses the following target components; 2-methyl-1,3-butadiene, 1-butanol, 3-methyl-1-butanol, 1-hexanol, 2-ethyl-1-hexanol, 1-propanol, 3-methyl-3-buten-1-ol, acetaldehyde, acetic acid, acetic acid methyl ester, acetone, acetonitrile, benzaldehyde, butyric acid, hexanal, isopropyl alcohol, methanol, nonanal, propanal, and 2-methyl-propionic acid. The assessment score is calculated by Formula (1). The standardized value calculating method, contribution deciding method, and reference score deciding method using Formula (1) are characterized as described earlier. The possibility of pancreatic cancer development is determined as high when the assessment score is greater than or equal to the reference score. The possibility of pancreatic cancer development is determined as low when the assessment scores in total are less than the reference score.

The possibility of pancreatic cancer being developed in test subjects was assessed and determined by the method 32. Then, all of 31 pancreatic cancer subjects were determined as having a high possibility of pancreatic cancer development, and all of 36 test subjects but pancreatic cancer subjects were determined as having a low possibility of pancreatic cancer development. The method 32 resulted in the sensitivity of 88% and the specificity of 100%. Thus, the method 32 has a very high sensitivity and an excellent specificity in determining the possibility of pancreatic cancer development.

<Assessment of possibility of pancreatic cancer development (assessment using relative concentrations and standardized values)>

In methods hereinafter described, the measurement unit 3 is a multireactive, real-time mass spectrometer. These methods compare different assessments; assessment using relative concentrations, assessment using standardized values, and assessment using neither of them.

(33-1) Method 33-1

A method 33-1 uses the following target components; 2-methyl butyric acid, butyric acid, propionic acid, methyl mercaptan, mercaptoacetone, trimethylamine, benzaldehyde, dimethyl sulfide, 2,3-butanedione, 3-hexanone, and 1-octen-3-ol. The concentration of each target component in the method 33-1 is a value obtained when the ratio of a target component included in collected exhaled air to the whole exhaled air is divided by the ratio of water vapor included in the exhaled air to the whole exhaled air.

The method 33-1 includes a first assessment and a second assessment. A determination result based on the first assessment takes precedence over the other.

The target components used for the first assessment are 2-methyl butyric acid, butyric acid, propionic acid, methyl mercaptan, mercaptoacetone, and trimethylamine. The threshold of each target component is a third quartile of concentrations of the target component in exhaled air of non-pancreatic cancer subjects. Each target component acquires 1 point when its concentration in exhaled air is greater than or equal to the threshold, and 0 point when the concentration is less than the threshold. The assessment score is the points in total of the target components. The first assessment uses two reference scores; a first reference score and a second reference score. The first reference score is 4 points. The possibility of pancreatic cancer development is determined as high when the assessment score is greater than or equal to the first reference score. The second reference score is 2 points. The possibility of pancreatic cancer development is determined as low when the assessment score is less than or equal to the second reference score. When the assessment score is less than the first reference score and greater than the second reference score (that is, the assessment score is 3 points), this method, without determining the possibility at this stage, proceeds to the second assessment.

The target components used for the second assessment are benzaldehyde, dimethyl sulfide, 2,3-butanedione, 3-hexanone, and 1-octen-3-ol. The threshold of each target component is a median of concentrations of the target component in exhaled air of non-pancreatic cancer subjects. Each target component acquires 1 point when its concentration in exhaled air is greater than or equal to the threshold, and 0 point when the concentration is less than the threshold. The assessment score is the points in total of the target components, and the reference score is 3 points. The possibility of pancreatic cancer development is determined as high when the assessment score is greater than or equal to the reference score. The possibility of pancreatic cancer development is determined as low when the assessment scores in total are less than the reference score.

The possibility of pancreatic cancer development was assessed and determined by the method 33-1 in 12 pancreatic cancer subjects and 28 non-pancreatic cancer subjects. This method resulted in the sensitivity of 75%, specificity of 86%, and accuracy (positive accuracy rate) of 83%.

(33-2) Method 33-2

The concentration of a target component in the method 33-2 is the ratio of the target component included in collected exhaled air to the whole exhaled air. Except that, the method 33-2 is consistent with the method 33-1.

The possibility of pancreatic cancer development was assessed and determined by the method 33-2 in 12 pancreatic cancer subjects and 28 non-pancreatic cancer subjects. This method resulted in the sensitivity of 42%, specificity of 86%, and accuracy (positive accuracy rate) of 73%.

The method 33-1 that uses relative concentrations was proven to have a higher sensitivity than the method 33-2 that dispenses with the use of relative concentrations.

(34-1) Method 34-1

A method 34-1 uses the following target components; methyl mercaptan, mercaptoacetone, dimethyl sulfide, dimethyl trisulfide, acetic acid, propionic acid, butyric acid, 2-methyl butyric acid, 2,3-butanedione, butanal, and hexanal. The concentration of each target component in the method 34-1 is a value obtained when the ratio of the target component included in collected exhaled air to the whole exhaled air is divided by the ratio of 2-butene included in the exhaled air to the whole exhaled air.

The method 34-1 includes a first assessment and a second assessment. A determination result based on the first assessment takes precedence over the other.

The target components used for the first assessment are methyl mercaptan, mercaptoacetone, dimethyl sulfide, dimethyl trisulfide, acetic acid, propionic acid, butyric acid, and 2-methyl butyric acid.

In the first assessment, the assessment score is calculated by the formulas 2) and 3) based on measurement results of concentrations of methyl mercaptan, mercaptoacetone, dimethyl sulfide, and dimethyl trisulfide. In Formula (3) according to this method, $\alpha_i$ is 1. The reference score is a third quartile of the assessment scores obtained with non-pancreatic cancer subjects.

In the first assessment, the assessment score is further calculated by the formulas 2) and 3) based on measurement results of concentrations of acetic acid, propionic acid, butyric acid, and 2-methyl butyric acid. In Formula (3) according to this method, $\alpha_i$ is 1. The reference score is a third quartile of the assessment scores obtained with non-pancreatic cancer subjects.

The possibility of pancreatic cancer development is determined as high when two different assessment scores obtained in the first assessment are both greater than or equal to the reference score. The possibility of pancreatic cancer development is determined as low when these two assessment scores are both less than the reference score. When one of the two assessment scores alone is greater than or equal to the reference score, this method, without determining the possibility at this stage, proceeds to the second assessment.

The target components used for the second assessment are 2,3-butanedione, butanal, and hexanal. In the second assessment, the assessment score is calculated by the formulas 2) and 3) based on concentration measurement results of these target components. In Formula (3) according to this method, $\alpha_i$ is 1. The reference score is a third quartile of the assessment scores obtained with non-pancreatic cancer subjects. The possibility of pancreatic cancer development is determined as high when the assessment score is greater than or equal to the reference score. The possibility of pancreatic cancer development is determined as low when the assessment score is less than the reference score.

The possibility of pancreatic cancer development was assessed and determined by the method 34-1 in 12 pancreatic cancer subjects and 29 non-pancreatic cancer subjects. This method resulted in the sensitivity of 83%, specificity of 79%, and accuracy (positive accuracy rate) of 80%.

(34-2) Method 34-2

The assessment score used in the method 34-2 is the sum of measurement results of target component concentrations. Except that, the method 34-2 is consistent with the method 34-1.

The possibility of pancreatic cancer development was assessed and determined by the method 34-2 in 12 pancreatic cancer subjects and 29 non-pancreatic cancer subjects. This method resulted in the sensitivity of 83%, specificity of 52%, and accuracy (positive accuracy rate) of 61%.

This demonstrates that a higher specificity is obtainable by the method 34-1 using assessment scores calculated by Formula (3) than the method 34-2 that dispenses with the use of assessment scores calculated by Formula (3).

(35-1) Method 35-1

A method 35-1 uses the following target components: dimethyl sulfide, acetic acid, and 2,3-butanedione. The concentration of a target component in the method 35-2 is the ratio of the target component included in exhaled air to the whole exhaled air.

The method 35-1 includes a first assessment and a second assessment. A determination result based on the first assessment takes precedence over the other.

The target components used for the first assessment are dimethyl sulfide and acetic acid. In the first assessment, concentrations of these target components are compared to their thresholds. The threshold of each target component is a third quartile of concentrations of the target component in exhaled air of non-pancreatic cancer subjects. Each target component acquires 1 point when its concentration is greater than or equal to the threshold, and 0 point when the concentration is less than the threshold. The assessment score is the points in total of the target components. The possibility of pancreatic cancer development is determined as high when the assessment score is 2 points. The possibility of pancreatic cancer development is determined as low when the assessment score is 0 point. When the assessment score is 1 point, this method, without determining the possibility at this stage, proceeds to the second assessment.

The target component used for the second assessment is 2,3-butanedione. Likewise, the concentration of this target component is compared to its threshold in the second assessment. The threshold of this target component is a third quartile of concentrations of the target component in exhaled air of non-pancreatic cancer subjects. The target component acquires 1 point when its concentration is greater than or equal to the threshold, and 0 point when the concentration is less than the threshold. The assessment score is the point of this target component. The possibility of pancreatic cancer development is determined as high when the assessment score is 1 point. The possibility of pancreatic cancer development is determined as low when the assessment score is 0 point.

The possibility of pancreatic cancer development was assessed and determined by the method 35-1 in 12 pancreatic cancer subjects and 29 non-pancreatic cancer subjects. This method resulted in the sensitivity of 8%, specificity of 86%, and accuracy (positive accuracy rate) of 63%.

(35-2) Method 35-2

The concentration of each target component in the method 35-2 is a value obtained when the ratio of the target component included in collected exhaled air to the whole exhaled air is divided by the ratio of water vapor included in the exhaled air to the whole exhaled air. Except that, the method 35-2 is consistent with the method 35-1.

The possibility of pancreatic cancer development was assessed and determined by the method 35-2 in 12 pancreatic cancer subjects and 29 non-pancreatic cancer subjects. This method resulted in the sensitivity of 83%, specificity of 79%, and accuracy (positive accuracy rate) of 80%.

(35-3) Method 35-3

A method 35-3 uses the following target components: dimethyl sulfide, acetic acid, and 2,3-butanedione. The concentration of each target component in the method 35-3 is a value obtained when the ratio of the target component included in collected exhaled air to the whole exhaled air is divided by the ratio of water vapor included in the exhaled air to the whole exhaled air.

The method 35-3 includes a first assessment and a second assessment. A determination result based on the first assessment takes precedence over the other.

The target components used for the first assessment are dimethyl sulfide and acetic acid. In the first assessment, standardized values of concentrations of these target components are compared to their thresholds. The standardized values are calculated by Formula (2). The threshold of each target component is a third quartile of concentration standardized values of the target component in exhaled air of non-pancreatic cancer subjects. Each target component acquires 1 point when its concentration standardized value is greater than or equal to the threshold, and 0 point when the concentration standardized value is less than the threshold. The assessment score is the points in total of the target components. The possibility of pancreatic cancer development is determined as high when the assessment score is 2 points. The possibility of pancreatic cancer development is determined as low when the assessment score is 0 point. When the assessment score is 1 point, this method, without determining the possibility at this stage, proceeds to the second assessment.

The target component used for the second assessment is 2,3-butanedione. Likewise, the concentration standardized value of this target component is compared to its threshold in the second assessment. The standardized value is calculated by Formula (2). The threshold of this target component is a third quartile of concentration standardized values of the target component in exhaled air of non-pancreatic cancer subjects. The target component acquires 1 point when its concentration standardized value is greater than or equal to the threshold, and 0 point when the concentration standardized value is less than the threshold. The assessment score is the point of this target component. The possibility of pancreatic cancer development is determined as high when the assessment score is 1 point. The possibility of pancreatic cancer development is determined as low when the assessment score is 0 point.

The possibility of pancreatic cancer development was assessed and determined by the method 35-3 in 12 pancreatic cancer subjects and 29 non-pancreatic cancer subjects. This method resulted in the sensitivity of 83%, specificity of 86%, and accuracy (positive accuracy rate) of 85%.

(36-1) Method 36-1

One of the target components of the method 35-1; dimethyl sulfide, was replaced with mercaptoacetone. Except that, the method 36-1 is consistent with the method 35-1,

The possibility of pancreatic cancer development was assessed and determined by the method 36-1 in 12 pancreatic cancer subjects and 29 non-pancreatic cancer subjects. This method resulted in the sensitivity of 50%, specificity of 59%, and accuracy (positive accuracy rate) of 56%.

(36-2) Method 36-2

One of the target components of the method 35-2; dimethyl sulfide, was replaced with mercaptoacetone. Except that, the method 36-2 is consistent with the method 35-2.
The possibility of pancreatic cancer development was assessed and determined by the method 36-2 in 12 pancreatic cancer subjects and 29 non-pancreatic cancer subjects. This method resulted in the sensitivity of 48%, specificity of 92%, and accuracy (positive accuracy rate) of 61%.

(36-3) Method 36-3

One of the target components of the method 35-3; dimethyl sulfide, was replaced with mercaptoacetone. Except that, the method 36-3 is consistent with the method 35-3.
The possibility of pancreatic cancer development was assessed and determined by the method 36-3 in 12 pancreatic cancer subjects and 29 non-pancreatic cancer subjects. This method resulted in the sensitivity of 83%, specificity of 79%, and accuracy (positive accuracy rate) of 80%.

(37-1) Method 37-1

One of the target components of the method 35-1; dimethyl sulfide, was replaced with dimethyl trisulfide. Except that, the method 37-1 is consistent with the method 35-1.
The possibility of pancreatic cancer development was assessed and determined by the method 37-1 in 12 pancreatic cancer subjects and 29 non-pancreatic cancer subjects. This method resulted in the sensitivity of 17%, specificity of 90%, and accuracy (positive accuracy rate) of 68%.

(37-2) Method 37-2

One of the target components of the method 37-2; dimethyl sulfide, was replaced with dimethyl trisulfide. Except that, the method 37-2 is consistent with the method 35-2.
The possibility of pancreatic cancer development was assessed and determined by the method 37-2 in 12 pancreatic cancer subjects and 29 non-pancreatic cancer subjects. This method resulted in the sensitivity of 92%, specificity of 76%, and accuracy (positive accuracy rate) of 80%.

(37-3) Method 37-3

One of the target components of the method 35-3; dimethyl sulfide, was replaced with dimethyl trisulfide. Except that, the method 37-3 is consistent with the method 35-3.
The possibility of pancreatic cancer development was assessed and determined by the method 37-3 in 12 pancreatic cancer subjects and 29 non-pancreatic cancer subjects. This method resulted in the sensitivity of 92%, specificity of 83%, and accuracy (positive accuracy rate) of 85%.

[Table 1]

|  | Method 1 | Method 2 | Method 3 | Method 4 | Method 5 |
|---|---|---|---|---|---|
| Type of determinable cancer | Breast cancer | Breast cancer | Breast cancer | Breast cancer | Breast cancer |
|  | 2-methyl-butyric acid | 2-methyl-butyric acid | 1-decanol | 3-methyl-1-butanol | 1-propanol |
|  | Benzaldehyde | Benzaldehyde | 3-methyl-1-butanol | Nonanal | 1-octen-3-ol |
|  | Decanal | Decanal | 1-hexanol | Benzaldehyde | Phenol |
|  |  | Phenol | 1-heptanal | Acetic acid | Heptanal |

(continued)

| | Method 1 | Method 2 | Method 3 | Method 4 | Method 5 |
|---|---|---|---|---|---|
| Type of determinable cancer | Breast cancer | Breast cancer | Breast cancer | Breast cancer | Breast cancer |
| Target component | | | α,α-dimethyl-β-phenylethyl=acetate | Ethyl acetate | Propionic acid |
| | | | 2-methyl-phenol | | 2-methyl-propionic acid |
| | | | 2-propenal | | Butyric acid |
| | | | Nonanal | | Acetoin |
| | | | Decanal | | Mercaptoacetone |
| | | | Benzaldehyde | | |
| | | | Acetic acid | | |
| | | | 2-methyl-butyric acid | | |
| | | | Ethyl acetate | | |
| | | | DL-alanyl-L-alanine | | |
| | | | 3,3-dimethyl-(3H) indazole | | |
| | | | 1-methylpyrrolidine | | |
| Sensitivity | 32% | 68% | 68% | 55% | 50% |
| Specificity | 100% | 97% | 100% | 97% | 90% |

[Table 2]

| | | Method 6 | Method 7 | Method 8 | Method 9 | Method 10 |
|---|---|---|---|---|---|---|
| Type of determinable cancer | | Breast cancer | Breast cancer | Breast cancer | Breast cancer | Breast cancer |
| Target component | | Acetoin | Butyric acid | 2-methyl-propionic acid | 2-methyl-propionic acid | 2-methyl-propionic acid |
| | | Butyric acid | Phenol | Acetic acid | Acetic acid | Acetic acid |
| | | 1-propanol | Trimethylamine | Butyric acid | Butyric acid | Butyric acid |
| | | Mercaptoacetone | Acetoin | Propionic acid | Propionic acid | Propionic acid |
| | | 1-methylpyrrolidine | Mercaptoacetone | Phenol | Trimethylamine | Phenol |
| | | 1-octen-3-ol | 1-octen-3-ol | Mercaptoacetone | 2-methylfuran | Benzaldehyde |
| | | | | 1-octen-3-ol | 1-propanol | |
| | | | | | Butanone | |
| | | | | | Acetoin | |
| Sensitivity | | 84% | 77% | 77% | 60% | 93% |
| Specificity | | 62% | 55% | 42% | 58% | 13% |

[Table 3]

| | Method 11 | Method 12 | Method 13 | Method 14 | Method 15 |
|---|---|---|---|---|---|
| Type of determinable cancer | Breast cancer | Breast cancer | Breast cancer | Pancreatic cancer | Pancreatic cancer |
| Target component | 2-methyl-propionic acid | 2-methyl-propionic acid | 2-methyl-propionic acid | 1-propanol | 1-propanol |
| | Acetic acid | Acetic acid | Acetic acid | 1,2-ethanediol | Phenol |
| | Butyric acid | Butyric acid | Butyric acid | Phenol | Heptanal |
| | Propionic acid | Propionic acid | Propionic acid | Propionic acid | Decanal |
| | Phenol | Phenol | Phenol | 2-methyl-propionic acid | Propionic acid |
| | Benzaldehyde | Benzaldehyde | Benzaldehyde | Butyric acid | 2-methyl-propionic acid |
| | | | | Heptanal | Butyric acid |
| | | | | Decanal | 2,3-butanedione |
| | | | | Butanal | Acetoin |
| | | | | 2,3-butanedione | |
| | | | | Acetoin | |
| Sensitivity | 87% | 70% | 60% | 55% | 19~32% |
| Specificity | 23% | 58% | 65% | 98% | 91~100% |

[Table 4]

| | | Method 16 | Method 17 | Method 18 | Method 19 | Method 20 |
|---|---|---|---|---|---|---|
| Type of determinable cancer | | Pancreatic cancer | Pancreatic cancer | Pancreatic cancer | Pancreatic cancer | Pancreatic cancer |
| Target component | | Phenol | Phenol | 1-methylpyrrolidine | 1-methylpyrrolidine | Trimethylamine |
| | | Butyric acid | Decanal | 2-propanol | Phenol | Decanal |
| | | Acetoin | 2,3-butanedione | 1-octen-3-ol | Heptanal | Acetophenone |
| | | Heptanal | Propionic acid | Heptanal | Decanal | Acetic acid |
| | | Decanal | 2-methyl-propionic acid | Decanal | Propionic acid | Mercaptoacetone |
| | | Carbon disulfide | Carbon disulfide | Propionic acid | 2-methyl-propionic acid | 2-methyl-propionic acid |
| | | 1-octen-3-ol | 1-octen-3-ol | 2-methyl-propionic acid | Butyric acid | 1-methylpyrrolidine |
| | | Benzaldehyde | Benzaldehyde | Butyric acid | Butanone | Phenol |
| | | Acetaldehyde | Acetaldehyde | Butanone | Acetoin | |
| | | Acetic acid | Acetic acid | Acetoin | | |
| | | 2-methylfuran | 2-methylfuran | | | |
| Sensitivity | | 39~58% | 16~48% | 26~39% | 19~32% | 67% |
| Specificity | | 84~100% | 48~98% | 93~96% | 91~100% | 89% |

## EP 3 351 934 A1

[Table 5]

|  | Method 21 | Method 22 | Method 23 | Method 24 | Method 25 |
|---|---|---|---|---|---|
| Type of determinable cancer | Pancreatic cancer | Pancreatic cancer | Pancreatic cancer | Pancreatic cancer | Pancreatic cancer |
| Target component | Trimethylamine | Trimethylamine | Propionic acid | Propionic acid | Propionic acid |
|  | Decanal | Acetophenone | 2-methyl-propionic acid | 2-methyl-propionic acid | 2-methyl-propionic acid |
|  | Acetophenone | 1-methylpyrrolidine | Butyric acid | Butyric acid | Butyric acid |
|  | Acetic acid | Mercaptoacetone | Acetic acid | Acetic acid | Acetic acid |
|  | Mercaptoacetone | 2-methylfuran | Acetophenone | Acetophenone | Acetophenone |
|  | 2-methyl-propionic acid | Phenol | Benzaldehyde | Benzaldehyde | Benzaldehyde |
|  | 1-methylpyrrolidine | 1-propanol |  |  | Trimethylamine |
|  | Phenol | Acetoin |  |  |  |
|  |  | Propionic acid |  |  |  |
|  |  | 2-methyl-propionic acid |  |  |  |
|  |  | Butyric acid |  |  |  |
|  |  | Acetic acid |  |  |  |
| Sensitivity | 67% | 27% | 52% | 67% | 67% |
| Specificity | 83% | 100% | 89% | 86% | 89% |

[Table 6]

|  | Method 26 | Method 27 | Method 28 | Method 29 | Method 30 |
|---|---|---|---|---|---|
| Type of determinable cancer | Pancreatic cancer | Pancreatic cancer | Pancreatic cancer | Pancreatic cancer | Pancreatic cancer |
| Target component | Acetophenone | Decanal | Decanal | Decanal | 1-octen-3-ol |
|  | Benzaldehyde | Butyric acid | Butyric acid | Butyric acid | 2-methylfuran |
|  | Phenol |  | 2-methyl-propionic acid | Trimethylamine | Decanal |
|  |  |  |  |  | Butyric acid |
|  |  |  |  |  | Trimethylamine |
| Sensitivity | 73% | 44% | 29% | 18% | 44% |
| Specificity | 71% | 93% | 96% | 100% | 96% |

[Table 7]

|  | Method 31 | Method 32 |
|---|---|---|
| Type of determinable cancer | Breast cancer | Pancreatic cancer |
|  | 1,2-ethanediol | 2-methyl-1,3-butadiene |
|  | Decanal | 1-butanol |

(continued)

|  | Method 31 | Method 32 |
|---|---|---|
| Type of determinable cancer | Breast cancer | Pancreatic cancer |
| Target component | Mercaptoacetone | 3-methyl-1-butanol |
|  | Heptanal | 1-hexanol |
|  | 6-methyl-5-hepten-2-one | 2-ethyl-1-hexanol |
|  | Cyclohexane | 1-propanol |
|  | Benzaldehyde | 3-methyl-3-buten-1-ol |
|  | 1-pentanol | Acetaldehyde |
|  | Decane | Acetic acid |
|  | 2,2,4-trimethyl-hexane | Acetic acid methyl ester |
|  | Dodecane | Acetone |
|  | 2-methyl-butyric acid | Acetonitrile |
|  | 1-butanol | Benzaldehyde |
|  | n-hexane | Butyric acid |
|  | 1-octen-3-ol | Hexanal |
|  | 4-ethylcyclohexanol | Isopropyl alcohol |
|  | Carbon disulfide | Nonanal |
|  | 2,4-bis(1,1-dimethylethyl)-phenol | Propanal |
|  | Nitrosomethylurethane | 2-methyl-propionic acid |
|  | Acetoin |  |
| Sensitivity | 79% | 88% |
| Specificity | 93% | 100% |

[Table 8]

| Type of determinable cancer | Method | | | | | | | | | | | | |
|---|---|---|---|---|---|---|---|---|---|---|---|---|---|
| | 33-1 | 33-2 | 34-1 | 34-2 | 35-1 | 35-2 | 35-3 | 36-1 | 36-2 | 36-3 | 37-1 | 37-2 | 37-3 |
| Type of determinable cancer | Pancreatic cancer | Pancreatic cancer | Pancreatic cancer | Pancreatic cancer | Pancreatic cancer | Pancreatic cancer | Pancreatic cancer | Pancreatic cancer | Pancreatic cancer | Pancreatic cancer | Pancreatic cancer | Pancreatic cancer | Pancreatic cancer |
| Target component | 2-methyl-butyric acid | | Methyl mercaptan | | Dimethyl sulfide | | | Mercaptoacetone | | | Dimethyl trisulfide | | |
| | Butyric acid | | Mercaptoacetone | | Acetic acid | | | Acetic acid | | | Acetic acid | | |
| | Propionic acid | | Dimethyl sulfide | | 2,3-butanedione | | | 2,3-butanedione | | | 2,3-butanedione | | |
| | Methyl mercaptan | | Dimethyl trisulfide | | | | | | | | | | |
| | Mercaptoacetone | | Acetic acid | | | | | | | | | | |
| | Trimethylamine | | Propionic acid | | | | | | | | | | |
| | Benzaldehyde | | Butyric acid | | | | | | | | | | |
| | Dimethyl sulfide | | 2-methyl-butyric acid | | | | | | | | | | |
| | 2,3-butanedione | | 2,3-butanedione | | | | | | | | | | |
| | 3-hexanone | | Butanal | | | | | | | | | | |
| | 1-octen-3-ol | | Hexanal | | | | | | | | | | |
| Sensitivity | 75% | 42% | 83% | 83% | 83% | 8% | 83% | 83% | 50% | 48% | 92% | 17% | 92% |
| Specificity | 86% | 86% | 79% | 52% | 86% | 86% | 79% | 79% | 59% | 92% | 83% | 90% | 76% |
| Accuracy | 83% | 73% | 80% | 61% | 85% | 63% | 80% | 80% | 56% | 61% | 85% | 68% | 80% |

The assessment device 1 structurally and technically characterized as described so far may readily provide in short time results of the determined possibility of cancer being developed in a test subject.

The assessment device for cancer development risk may be particularly useful in determining the possibility of breast cancer or pancreatic cancer being developed in a test subject. The assessment device for cancer development risk may be considered applicable to cases that need to determine the possibility of other cancers being developed, for example, lung cancer, stomach cancer, and esophageal cancer.

As is obvious from the embodiments described thus far, the assessment device for cancer development risk according to the first aspect includes a measurement unit and a controller. The measurement unit is configured to measure concentrations of a plurality of types of target components included in exhaled air collected from a subject to be assessed. The controller is configured to calculate an assessment score correlated to the possibility of cancer being developed in the subject to be assessed based on measurement results of the concentrations obtained by the measurement unit.

The first aspect may allow the possibility of cancer being developed in a human subject to be readily determined in short time based on the assessment score.

In the second aspect, the assessment device for cancer development risk according to the first aspect is further characterized in that the concentrations are each a relative concentration calculated on the basis of the ratio of any one component but the target components included in the exhaled air.

The second aspect may allow the possibility of cancer development to be assessed with improved accuracy.

In the third aspect, the assessment device for cancer development risk according to the second aspect is further characterized in that the any one component is water vapor or 2-butene.

The third aspect may allow the possibility of cancer development to be more accurately assessed.

In the fourth aspect, the assessment device for cancer development risk according to any one of the first to third aspects is further characterized in that the controller is configured to determine a high possibility of cancer being developed in the subject to be assessed when the assessment score is equal to a certain value or within a certain range of values.

The fourth aspect may allow the possibility of cancer being developed in a human subject to be readily determined in short time.

In the fifth aspect, the assessment device for cancer development risk according to any one of the first to fourth aspects is further characterized in that the controller is configured to calculate the assessment score based on results obtained by comparing the measurement results of the concentrations to thresholds respectively set for the plurality of types of target components.

The fifth aspect may allow the possibility of cancer development to be even more accurately assessed.

In the sixth aspect, the assessment device for cancer development risk according to any one of the first to fourth aspects is further characterized in that the controller is configured to standardize the measurement results of the concentrations to calculate a plurality of standardized values and then calculate the assessment score based on results obtained by comparing measurement results of the standardized values to thresholds respectively set for the plurality of types of target components.

The sixth aspect may allow the possibility of cancer development to be even more accurately assessed.

In the seventh aspect, the assessment device for cancer development risk according to any one of the first to fourth aspects is further characterized in that the controller is configured to standardize the measurement results of the concentrations to calculate a plurality of standardized values and then calculate the assessment score based on the plurality of standardized values and levels of contribution of the plurality of standardized values.

The seventh aspect may allow the possibility of cancer development to be even more accurately assessed.

In the eight aspect, the assessment device for cancer development risk according to the seventh aspect is further characterized in that the assessment score is calculated based on combination of two or more of the plurality of standardized values and a level of contribution of the combination.

The eighth aspect may allow the possibility of cancer development to be even more accurately assessed.

In the ninth aspect, the assessment device for cancer development risk according to the seventh aspect is further characterized in that the controller is configured to calculate the assessment score using Formula (1).

$$S = \sum_{i=1}^{n} \sum_{j=1}^{n} \frac{k_{ij} A_i A_j}{n} \quad \ldots(1)$$

In Formula (1), n is the number of types of target components, $A_i$ is the standardized value of an i(th) target component, $A_j$ is the standardized value of a j(th) target component, and $k_{ij}$ is the level of contribution of combination of the standardized values of the i(th) and j(th) target components, and S is the assessment score.

The ninth aspect may allow the possibility of cancer development to be even more accurately assessed.

In the tenth aspect, the assessment device for cancer development risk according to any one of the first to fourth aspects is further characterized in that the controller is configured to calculate the assessment score using Formula (3).

$$S = \sum_{i=1}^{n} A_i \times \alpha_i \quad \dots (3)$$

In Formula (3), n is the number of types of target components, $A_i$ is the standardized value of an i(th) target component, $\alpha_i$ is the level of contribution of the standardized value of the i(th) target component, and S is the assessment score.

The tenth aspect may allow the possibility of cancer development to be even more accurately assessed.

In the eleventh aspect, the assessment device for cancer development risk according to any one of the first to tenth aspects is further characterized in that the plurality of types of target components include at least two types of components selected from the group consisting of 1-methylpyrrolidine, 2-methyl-propionic acid, benzaldehyde, acetic acid, butyric acid, phenol, trimethylamine, 1-octen-3-ol, mercaptoacetone, acetophenone, acetoin, carbon disulfide, 2-methyl-butyric acid, 6-methyl-5-hepten-2-one, decanal, propionic acid, heptanal, 2-methylfuran, 2,3-butanedione, nonanal, butanal, hexanoic acid, methyl mercaptan, dimethyl sulfide, and dimethyl trisulfide.

The eleventh aspect may allow the possibility of cancer development to be even more accurately assessed.

In the twelfth aspect, the assessment device for cancer development risk according to any one of the first to tenth aspects is further characterized in that the plurality of types of target components include at least two types of components selected from the group consisting of 1-methylpyrrolidine, carbon disulfide, acetaldehyde, 2-methyl-butyric acid, acetone, 6-methyl-5-hepten-2-one, 2-methyl-propionic acid, decanal, benzaldehyde, acetic acid, butyric acid, 1-propanol, propionic acid, 1-heptanol, heptanal, 2-methoxy-1-propanol, phenol, trimethylamine, 1-octen-3-ol, mercaptoacetone, 2-butanone, acetophenone, 2-methylfuran, acetoin, 2,3-butanedione, methanol, cyclohexanone, dimethyl trisulfide, 2,4-dimethylhexane, 1-pentanol, n-hexane, 1-heptene, 4-ethylcyclohexanol, acetic acid methyl ester, nitrosomethylurethane, 2-methyl-1,3-butadiene, decane, 3-methyl-1-butanol, dodecane, 2-ethyl-1-hexanol, 2,2,4-trimethylhexane, 3-methyl-3-buten-1-ol, acetonitrile, 2,4-bis(1,1-dimethylethyl)-phenol, hexanal, nonane, nonanal, butanal, 2,4-dimethylheptane, hexanoic acid, 1-octene, 1,2-ethanediol, 1-pentene, butylbenzene, 5-methyl-1-heptene, ethylacetate, 7,8-dioxabicyclo[3.2.1]oct-2-ene, o-xylene, 1-hexanol, β-phellandrene, menthol, 2-propenal, propanal, D-limonene, dimethyl disulfide, 1-methoxy-2-propanol, 2-butene, octanal, 4-isopropenyltoluene, 1,3-dichlorobenzene, 1,2,4-trimethylbenzene, 1-butanol, methyl mercaptan, and dimethyl sulfide.

The twelfth aspect may allow the possibility of cancer development to be even more accurately assessed.

In the thirteenth aspect, the assessment device for cancer development risk according to any one of the first to tenth aspects is further characterized in that the plurality of types of target components include at least two types of components selected from the group consisting of benzaldehyde, heptanal, acetic acid, 2-methyl-butyric acid, 6-methyl-5-hepten-2-one, 2-methyl-propionic acid, decanal, phenol, butanal, butyric acid, 1-octen-3-ol, 1-heptanol, 2-methyl-1-propanol, 1-propanol, acetoin, propionic acid, 2-butanone, 2,3-butanedione, mercaptoacetone, trimethylamine, acetophenone, cyclohexanone, 1,2-ethanediol, 1-pentanol, 1-butanol, 4-ethylcyclohexanol, nitrosomethylurethane, decane, dodecane, 2,2,4-trimethylhexane, n-hexane, 2,4-bis(1,1-dimethylethyl)-phenol, nonane, 3-methyl-1-butanol, 2,4-dimethylheptane, 1-octene, 2-methylfuran, nonanal, butylbenzene, ethylacetate, o-xylene, β-phellandrene, 2-propenal, D-limonene, 1-methoxy-2-propanol, octanal, and 1,3-dichlorobenzene.

The thirteenth aspect may allow the possibility of pancreatic cancer development to be more accurately assessed.

In the fourteenth aspect, the assessment device for cancer development risk according to any one of the first to tenth aspects is further characterized in that the plurality of types of target components include at least two types of components selected from the group consisting of 1-methylpyrrolidine, carbon disulfide, acetaldehyde, acetone, 2-methyl-propionic acid, decanal, benzaldehyde, acetic acid, butyric acid, 1-propanol, propionic acid, heptanal, phenol, trimethylamine, 1-octen-3-ol, mercaptoacetone, acetophenone, 2-methylfuran, 6-methyl-5-hepten-2-one, acetoin, 2,3-butanedione, methanol, dimethyl trisulfide, 2,4-dimethyl-hexane, n-hexane, 1-heptene, acetic acid methyl ester, 2-methyl-1,3-butadiene, 3-methyl-1-butanol, 2-ethyl-1-hexanol, 3-methyl-3-buten-1-ol, acetonitrile, hexanal, nonanal, butanal, hexanoic acid, 1,2-ethanediol, 2,4-bis(1,1-dimethylethyl)-phenol, 1-pentene, 5-methyl-1-heptene, 7,8-dioxabicyclo[3.2.1]oct-2-ene, 1-hexanol, menthol, propanal, dimethyl disulfide, 2-butene, 4-isopropenyltoluene, 1,2,4-trimethylbenzene, 1-butanol, methyl mercaptan, hexanone, and dimethyl sulfide.

The fourteenth aspect may allow the possibility of breast cancer development to be more accurately assessed.

In the fifteenth aspect, the assessment device for cancer development risk according to any one of the first to

EP 3 351 934 A1

thirteenth aspects is further characterized in that the assessment score correlates to the possibility of breast cancer being developed in the subject to be assessed.

The fifteenth aspect may allow the possibility of breast cancer development to be even more accurately assessed.

In the sixteenth aspect, the assessment device for cancer development risk according to any one of the first to twelfth and fourteenth aspects is further characterized in that the assessment score correlates to the possibility of pancreatic cancer being developed in the subject to be assessed.

The sixteenth aspect may allow the possibility of pancreatic cancer development to be even more accurately assessed.

A program according to the seventeenth aspect is for use in prompting a computer to effectuate functions of the controller provided in the assessment device for cancer development risk according to any one of claims 1 to 16.

The seventeenth aspect may allow the possibility of cancer development in a human subject to be readily determined in short time by the use of the program.

A testing method for cancer development risk according to the eighteenth aspect includes steps of: (a) collecting exhaled air from a subject to be assessed; (b) measuring concentrations of a plurality of types of target components included in the exhaled air; and (c) calculating an assessment score from measurement results of the concentrations and comparing the assessment score to a reference score based on a criterion that cancer is being developed in the subject to be assessed with a high possibility when the assessment score is equal to a certain value or within a certain range of values.

The eighteenth aspect may allow the possibility of cancer development in a human subject to be readily determined in short time.

DESCRIPTION OF REFERENCE SIGNS

1    Assessment device for cancer development risk
3    Measurement unit
4    Controller

**Claims**

1. A cancer development risk assessment device, comprising:

   a measurement unit configured to measure concentrations of a plurality of types of target components included in exhaled air collected from a subject to be assessed; and
   a controller configured to calculate an assessment score correlated to the possibility of cancer being developed in the subject to be assessed based on measurement results of the concentrations obtained by the measurement unit.

2. The cancer development risk assessment device according to claim 1, wherein
   the concentrations are each a relative concentration calculated on the basis of a ratio of any one component but the target components included in the exhaled air.

3. The cancer development risk assessment device according to claim 2, wherein
   the any one component is water vapor or 2-butene.

4. The cancer development risk assessment device according to any one of claims 1 to 3, wherein
   the controller is configured to determine a high possibility of cancer being developed in the subject to be assessed when the assessment score is equal to a certain value or within a certain range of values.

5. The cancer development risk assessment device according to any one of claims 1 to 4, wherein
   the controller is configured to calculate the assessment score based on results obtained by comparing the measurement results of the concentrations to thresholds respectively set for the plurality of types of target components.

6. The cancer development risk assessment device according to any one of claims 1 to 4, wherein
   the controller is configured to standardize the measurement results of the concentrations to calculate a plurality of

40

standardized values and then calculate the assessment score based on results obtained by comparing measurement results of the standardized values to thresholds respectively set for the plurality of types of target components.

7.  The cancer development risk assessment device according to any one of claims 1 to 4, wherein the controller is configured to standardize the measurement results of the concentrations to calculate a plurality of standardized values and then calculate the assessment score based on the plurality of standardized values and levels of contribution of the plurality of standardized values.

8.  The cancer development risk assessment device according to claim 7, wherein the assessment score is calculated based on combination of two or more of the plurality of standardized values and a level of contribution of the combination.

9.  The cancer development risk assessment device according to claim 7, wherein the controller is configured to calculate the assessment score using the following formula (1),

$$S = \sum_{i=1}^{n}\sum_{j=1}^{n}\frac{k_{ij}A_iA_j}{n} \quad \ldots(1)$$

where n is the number of types of target components, $A_i$ is the standardized value of an i(th) target component, $A_j$ is the standardized value of a j(th) target component, $k_{ij}$ is a level of contribution of combination of the standardized values of the i(th) and j(th) target components, and S is the assessment score.

10. The cancer development risk assessment device according to any one of claims 1 to 4, wherein the controller is configured to calculate the assessment score using the following formula (3),

$$S = \sum_{i=1}^{n} A_i \times \alpha_i \quad \ldots(3)$$

where n is the number of types of target components, $A_i$ is the standardized value of an i(th) target component, $\alpha_i$ is a level of contribution of the standardized value of the i(th) target component, and S is the assessment score.

11. The cancer development risk assessment device according to any one of claims 1 to 10, wherein the plurality of types of target components comprise at least two types of components selected from the group consisting of 1-methylpyrrolidine, 2-methyl-propionic acid, benzaldehyde, acetic acid, butyric acid, phenol, trimethylamine, 1-octen-3-ol, mercaptoacetone, acetophenone, acetoin, carbon disulfide, 2-methyl-butyric acid, 6-methyl-5-hepten-2-one, decanal, propionic acid, heptanal, 2-methylfuran, 2,3-butanedione, nonanal, butanal, hexanoic acid, methyl mercaptan, dimethyl sulfide, and dimethyl trisulfide.

12. The cancer development risk assessment device according to any one of claims 1 to 10, wherein the plurality of types of target components comprise at least two types of components selected from the group consisting of 1-methylpyrrolidine, carbon disulfide, acetaldehyde, 2-methyl-butyric acid, acetone, 6-methyl-5-hepten-2-one, 2-methyl-propionic acid, decanal, benzaldehyde, acetic acid, butyric acid, 1-propanol, propionic acid, 1-heptanol, heptanal, 2-methoxy-1-propanol, phenol, trimethylamine, 1-octen-3-ol, mercaptoacetone, 2-butanone, acetophenone, 2-methylfuran, acetoin, 2,3-butanedione, methanol, cyclohexanone, dimethyl trisulfide, 2,4-dimethylhexane, 1-pentanol, n-hexane, 1-heptene, 4-ethylcyclohexanol, acetic acid methyl ester, nitrosomethylurethane, 2-methyl-1,3-butadiene, decane, 3-methyl-1-butanol, dodecane, 2-ethyl-1-hexanol, 2,2,4-trimethylhexane, 3-methyl-3-buten-1-ol, acetonitrile, 2,4-bis(1,1-dimethylethyl)-phenol, hexanal, nonane, nonanal, butanal, 2,4-dimethylheptane, hexanoic acid, 1-octene, 1,2-ethanediol, 1-pentene, butylbenzene, 5-methyl-1-heptene, ethylacetate, 7,8-dioxabicyclo[3.2.1]oct-2-ene, o-xylene, 1-hexanol, β-phellandrene, menthol, 2-propenal, propanal, D-limonene, dimethyl disulfide, 1-methoxy-2-propanol, 2-butene, octanal, 4-isopropenyltoluene, 1,3-dichlorobenzene, 1,2,4-trimethylbenzene, 1-butanol, methyl mercaptan, and dimethyl sulfide.

13. The cancer development risk assessment device according to any one of claims 1 to 10, wherein the plurality of types of target components comprise at least two types of components selected from the group consisting of benzaldehyde, heptanal, acetic acid, 2-methyl-butyric acid, 6-methyl-5-hepten-2-one, 2-methyl-propionic acid, decanal, phenol, butanal, butyric acid, 1-octen-3-ol, 1-heptanol, 2-methyl-1-propanol, 1-propanol, acetoin, propionic acid, 2-butanone, 2,3-butanedione, mercaptoacetone, trimethylamine, acetophenone, cyclohexanone, 1,2-ethanediol, 1-pentanol, 1-butanol, 4-ethylcyclohexanol, nitrosomethylurethane, decane, dodecane, 2,2,4-trimethylhexane, n-hexane, 2,4-bis(1,1-dimethylethyl)-phenol, nonane, 3-methyl-1-butanol, 2,4-dimethylheptane, 1-octene, 2-methylfuran, nonanal, butylbenzene, ethylacetate, o-xylene, β-phellandrene, 2-propenal, D-limonene, 1-methoxy-2-propanol, octanal, and 1,3-dichlorobenzene.

14. The cancer development risk assessment device according to any one of claims 1 to 10, wherein
the plurality of types of target components comprise at least two types of components selected from the group consisting of 1-methylpyrrolidine, carbon disulfide, acetaldehyde, acetone, 2-methyl-propionic acid, decanal, benzaldehyde, acetic acid, butyric acid, 1-propanol, propionic acid, heptanal, phenol, trimethylamine, 1-octen-3-ol, mercaptoacetone, acetophenone, 2-methylfuran, 6-methyl-5-hepten-2-one, acetoin, 2,3-butanedione, methanol, dimethyl trisulfide, 2,4-dimethyl-hexane, n-hexane, 1-heptene, acetic acid methyl ester, 2-methyl-1,3-butadiene, 3-methyl-1-butanol, 2-ethyl-1-hexanol, 3-methyl-3-buten-1-ol, acetonitrile, hexanal, nonanal, butanal, hexanoic acid, 1,2-ethanediol, 2,4-bis(1,1-dimethylethyl)-phenol, 1-pentene, 5-methyl-1-heptene, 7,8-dioxabicyclo[3.2.1]oct-2-ene, 1-hexanol, menthol, propanal, dimethyl disulfide, 2-butene, 4-isopropenyltoluene, 1,2,4-trimethylbenzene, 1-butanol, methyl mercaptan, hexanone, and dimethyl sulfide.

15. The cancer development risk assessment device according to any one of claims 1 to 13, wherein the assessment score correlates to the possibility of breast cancer being developed in the subject to be assessed.

16. The cancer development risk assessment device according to any one of claims 1 to 12 and 14, wherein the assessment score correlates to the possibility of pancreatic cancer being developed in the subject to be assessed.

17. A program for use in prompting a computer to effectuate functions of the controller provided in the cancer development risk assessment device according to any one of claims 1 to 16.

18. A method for testing cancer development risk, comprising steps of:

(a) collecting exhaled air from a subject to be assessed;
(b) measuring concentrations of a plurality of types of target components included in the exhaled air; and
(c) calculating an assessment score from measurement results of the concentrations, and comparing the assessment score to a certain value or to a certain range of values based on a criterion that cancer is being developed in the subject to be assessed with a high possibility when the assessment score is equal to the certain value or within the certain range of values.

# FIG. 1

# FIG. 2

# FIG. 3

## INTERNATIONAL SEARCH REPORT

| International application No. |
| --- |
| PCT/JP2016/004374 |

A. CLASSIFICATION OF SUBJECT MATTER
*G01N33/497(2006.01)i, G01N33/48(2006.01)i*

According to International Patent Classification (IPC) or to both national classification and IPC

B. FIELDS SEARCHED

Minimum documentation searched (classification system followed by classification symbols)
G01N33/497, G01N33/48

Documentation searched other than minimum documentation to the extent that such documents are included in the fields searched
Jitsuyo Shinan Koho          1922-1996    Jitsuyo Shinan Toroku Koho    1996-2016
Kokai Jitsuyo Shinan Koho    1971-2016    Toroku Jitsuyo Shinan Koho    1994-2016

Electronic data base consulted during the international search (name of data base and, where practicable, search terms used)
CAplus/REGISTRY/MEDLINE/EMBASE/BIOSIS(STN), PubMed

C. DOCUMENTS CONSIDERED TO BE RELEVANT

| Category* | Citation of document, with indication, where appropriate, of the relevant passages | Relevant to claim No. |
| --- | --- | --- |
| X<br>Y | US 2011/0269632 A1  (TECHNION RESEARCH AND DEVELOPMENT FOUNDATION LTD.),<br>03 November 2011 (03.11.2011),<br>entire text; all drawings<br>& WO 2010/079490 A1    & EP 2376913 A1 | 1,4,12-18<br>2-3,5-11 |
| X<br>Y<br>A | WO 2015/031617 A1  (UNIVERSITY OF LOUISVILLE RESEARCH FOUNDATION, INC.),<br>05 March 2015 (05.03.2015),<br>paragraphs [0090] to [0094]<br>& US 2015/0064796 A1    & EP 3039428 A1 | 1,4,17-18<br>2-3,5-15<br>16 |
| X<br>Y<br>A | US 2014/0127326 A1  (SOOD Anil K.),<br>08 May 2014 (08.05.2014),<br>paragraphs [0088] to [0091]<br>& WO 2012/122128 A2 | 1,4,17-18<br>2-3,5-15<br>16 |

☒ Further documents are listed in the continuation of Box C.      ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>25 November 2016 (25.11.16) | Date of mailing of the international search report<br>06 December 2016 (06.12.16) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

<div align="center">

**INTERNATIONAL SEARCH REPORT**

</div>

| International application No. |
| --- |
| PCT/JP2016/004374 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2015-526732 A  (Automotive Coalition for Traffic Safety, Inc.), 10 September 2015 (10.09.2015), paragraph [0025] & US 2015/0233897 A1 paragraph [0025] & WO 2014/031071 A1     & EP 2888588 A1 | 2-3 |
| Y | JP 2015-507205 A  (BG Medicine, Inc.), 05 March 2015 (05.03.2015), claim 1; example 1 & US 2013/0244892 A1 claim 1; example 1 & WO 2013/120114 A1     & EP 2812703 A1 | 5-10 |
| Y | KUMAR S et al., Selected ion flow tube mass spectrometry analysis of exhalted breath for volatile organic compound profiling of esophago-gastric cancer, Anal Chem., 2013.06.18, 85(12), 6121-6128 | 11-14 |
| Y | BUSZEWSKI B, Identification of volatile lung cancer markers by gas chromatography-mass spectrometry: comparison with discrimination by canines, Anal bioanal Chem., 2012.07, 404(1), 141-146 | 11-14 |
| Y | JP 2002-534697 A  (PHILLIPS, Michael), 15 October 2002 (15.10.2002), claims & US 6221026 B1           & WO 2000/041623 A1 claims & EP 1150606 A1 | 12-15 |

Form PCT/ISA/210 (continuation of second sheet) (January 2015)

**REFERENCES CITED IN THE DESCRIPTION**

**Patent documents cited in the description**

• JP 2009518654 A